# EUROPEAN PATENT APPLICATION

(11) **EP 4 559 390 A2**
(43) Date of publication of application: **28.05.2025**
(21) Application number: 25156440.7
(22) Date of filing: 29.01.2020
(51) Int. Cl.: A61B 5/11

(54) **DIGITAL BIOMARKER**

(30) Priority: 31.01.2019 EP 19154742
(62) Divisional of application: 20702984.4
(71) Applicant: F. Hoffmann-La Roche AG, 4070 Basel (CH)
(72) Inventor: GOSSENS, Christian, 4070 Basel (CH); LINDEMANN, Michael, 4051 Basel (CH); LIPSMEIER, Florian, 4070 Basel (CH)
(74) Representative: Jenni, Wolfgang

(57) **Abstract**

Currently, assessing the severity and progression of symptoms in a patient diagnosed with Huntington's disease involves in-clinic monitoring and testing of the patient every 6 to 12 months. However, monitoring and testing a patient more frequently is preferred, but increasing the frequency of in-clinic monitoring and testing can be costly and inconvenient to the patient. Thus, assessing the severity and progression of symptoms via remote monitoring and testing of the patient outside of a clinic environment as described herein provides advantages in cost, ease of monitoring and convenience to the patient. Systems, methods and devices according to the present disclosure provide a diagnostic for assessing one or more symptoms of Huntington's disease in a patient by passive monitoring of the patient and/or active testing of the patient.

## Description

### FIELD

Aspects described herein generally relate to medical diagnostics and medical devices for improved patient testing and patient analysis. More specifically, aspects described herein provide diagnostic devices, systems and methods for assessing symptom severity and progression of Huntington's disease in a patient by active testing and/or passive monitoring of the patient.

### BACKGROUND

Huntington's disease is an inherited condition that leads to the progressive degeneration of nerve cells in the brain. A diagnosis of Huntington's disease may be based on neurological testing, genetic testing, and imaging, as well as family history and symptoms. The disease causes a wide variety of symptoms associated with motor function, cognitive function, behavioral function and functional capacity of the patient. Symptoms associated with motor function can include both involuntary movement problems and impairments in voluntary movements, such as involuntary jerking or writhing movements (chorea), muscle problems, such as rigidity or muscle contracture (dystonia), slow or abnormal eye movements, impaired gait, posture and balance, difficulty with the physical production of speech or swallowing. Impairments in voluntary movements can impact a person's ability to work, perform daily activities, communicate and remain independent. Although there is no known cure, treatments such as medications, therapies, and life style changes can help the patient cope with the symptoms of the disease. Additionally, the onset, severity and progression of the symptoms of Huntington's disease can vary between individuals. Thus, early detection of even small changes in the severity and progression of symptoms is important for guiding treatment and therapy options.

There are several standardized methods and tests for measuring the symptom severity and progression in patients diagnosed with Huntington's disease. Each of the tests involves a doctor measuring the subject's abilities to perform various mental and physical functions in different ways. These standardized tests can provide an assessment of the various symptoms associated with the patient's cognitive, behavioral, motor functions and capabilities and can help track changes in these symptoms over time. Assessing symptom severity and progression using standardized methods and tests can, therefore, help guide treatment and therapy options.

Currently, assessing the severity and progression of symptoms in a patient diagnosed with Huntington's disease involves in-clinic monitoring and testing of the patient every 6 to 12 months. While monitoring and testing a patient more frequently is ideal, increasing the frequency of in-clinic monitoring and testing can be costly and inconvenient to the patient.

### BRIEF SUMMARY

The following presents a simplified summary of various aspects described herein. This summary is not an extensive overview, and is not intended to identify key or critical elements or to delineate the scope of the claims. The following summary merely presents some concepts in a simplified form as an introductory prelude to the more detailed description provided below. Aspects described herein describe specialized medical devices for assessing the severity and progression of symptoms for a patient diagnosed with Huntington's disease. Testing and monitoring may be done remotely and outside of a clinic environment, thereby providing lower cost, increased frequency, and simplified ease and convenience to the patient, resulting in improved detection of symptom progression, which in turn results in better treatment.

According to one aspect, the disclosure relates to a diagnostic device for assessing one or more symptoms of Huntington's disease in a subject. The device includes at least one processor, one or more sensors associated with the device, and memory storing computer-readable instructions that, when executed by the at least one processor, cause the device to receive a plurality of first sensor data via the one or more sensors associated with the device, extract, from the received first sensor data, a first plurality of features associated with the one or more symptoms of Huntington's disease in the subject, and determine a first assessment of the one or more symptoms of Huntington's disease based on the extracted first plurality of features.

A certain embodiment of the invention relates to a diagnostic device for assessing one or more symptoms of Huntington's disease in a subject, the device comprising:
- at least one processor;
- one or more sensors associated with the device; and
- memory storing computer-readable instructions that, when executed by the at least one processor, cause the device to:
   ∘receive a plurality of first sensor data via the one or more sensors associated with the device;
   ∘extract, from the received first sensor data, a first plurality of features associated with the one or more symptoms of Huntington's disease in the subject; and
   ∘determine a first assessment of the one or more symptoms of Huntington's disease based on the extracted first plurality of features.

A certain embodiment of the invention relates to the device as described herein, wherein the computer-readable instructions, when executed by the at least one processor, further cause the device to:
- prompt the subject to perform one or more diagnostic tasks;
- in response to the subject performing the one or more diagnostic tasks, receive a plurality of second sensor data via the one or more sensors associated with the device;
- extract, from the received second sensor data, a second plurality of features associated with the one or more symptoms of Huntington's disease; and
- determine a second assessment of the one or more symptoms of Huntington's disease based on the extracted second plurality of features.

A certain embodiment of the invention relates to the device as described herein, wherein the one or more symptoms of Huntington's disease in the subject include at least one of a symptom indicative of a cognitive function of the subject, a symptom indicative of a motor function of the subject, a symptom indicative of a behavioral function of the subject, or a symptom indicative of a functional capacity of the subject.

A certain embodiment of the invention relates to the device as described herein, wherein the one or more symptoms of Huntington's disease in the subject include at least one of a symptom indicative of a cognitive function of the subject, a symptom indicative of a motor function of the subject, a symptom indicative of a behavioral function of the subject, or a symptom indicative of a functional capacity of the subject, whereby the patient mobility is assessed at least partly based on GPS location data.

A certain embodiment of the invention relates to the device as described herein, wherein the one or more symptoms of Huntington's disease in the subject are indicative of at least one of visuo-motor integration, visual attention, motor speed, cognitive processing speed, chorea, dystonia, visuo-motor coordination, fine motor impairment, upper-body or lower-body bradykinesia.

A certain embodiment of the invention relates to the device as described herein, wherein the one or more sensors associated with the device comprise at least one of a first sensor disposed within the device or a second sensor worn by the subject and configured to communicate with the device.

A certain embodiment of the invention relates to the device as described herein, wherein prompting the subject to perform the one or more diagnostic tasks includes at least one of prompting the subject to answer one or more questions or prompting the subject to perform one or more actions.

A certain embodiment of the invention relates to the device as described herein, wherein the one or more diagnostic tasks are associated with at least one of a EQ-5D-5L test, WPAI-HD test, HD-SDI test, speed tapping test, draw a shape test, chorea test, balance test, u-turn test, SDMT test, and word reading test.

A certain embodiment of the invention relates to a computer-implemented method for assessing one or more symptoms of Huntington's disease in a subject, the method comprising:
- receiving a plurality of first sensor data via one or more sensors associated with a device;
- extracting, from the received first sensor data, a first plurality of features associated with the one or more symptoms of Huntington's disease in the subject; and
- determining a first assessment of the one or more symptoms of Huntington's disease based on the extracted first plurality of features.

A certain embodiment of the invention relates to the computer-implemented method as described herein, further comprising:
- prompting the subject to perform one or more diagnostic tasks;
- in response to the subject performing the one or more diagnostics tasks, receiving, a plurality of second sensor data via the one or more sensors;
- extracting, from the received second sensor data, a second plurality of features associated with one or more symptoms of Huntington's disease; and
- determining a second assessment of the one or more symptoms of Huntington's disease based on at least the extracted second sensor data.

A certain embodiment of the invention relates to the computer-implemented method as described herein, wherein the one or more symptoms of Huntington's disease in the subject include at least one of a symptom indicative of a cognitive function of the subject, a symptom indicative of a motor function of the subject, a symptom indicative of a behavioral function of the subject, or a symptom indicative of a functional capacity of the subject, in particular wherein the one or more symptoms of Huntington's disease in the subject are indicative of at least one of visuo-motor integration, visual attention, motor speed, cognitive processing speed, chorea, dystonia, visuo-motor coordination, fine motor impairment, upper-body or lower-body bradykinesia.

A certain embodiment of the invention relates to the computer-implemented method as described herein, whereby the patient mobility is assessed at least partly based on GPS location data.

A certain embodiment of the invention relates to the computer-implemented method as described herein, wherein the one or more sensors associated with the device comprise at least one of a first sensor disposed within the device or a second sensor located on the subject and configured to communicate with the device, in particular wherein prompting the subject to perform the one or more diagnostic tasks includes at least one of prompting the subject answer one or more questions or prompting the subject to perform one or more actions.

A certain embodiment of the invention relates to the computer-implemented method as described herein, wherein the one or more diagnostic tasks are associated with at least one of a EQ-5D-5L test, WPAI-HD test, HD-SDI test, speed tapping test, draw a shape test, chorea test, balance test, u-turn test, SDMT test, and word reading test.

A certain embodiment of the invention relates to a non-transitory machine readable storage medium comprising machine-readable instructions for causing a processor to execute a method for assessing one or more symptoms of Huntington's disease in a subject, the method comprising:
- receiving a plurality of sensor data via one or more sensors associated with a device;
- extracting, from the received sensor data, a plurality of features associated with the one or more symptoms of Huntington's disease in a subject; and
- determining an assessment of the one or more symptoms of Huntington's disease based on the extracted plurality of features.

In some embodiments, the computer-readable instructions, when executed by the at least one processor, further cause the device to prompt the subject to perform one or more diagnostic tasks, in response to the subject performing the one or more diagnostic tasks, receive a plurality of second sensor data via the one or more sensors associated with the device, extract, from the received second sensor data, a second plurality of features associated with the one or more symptoms of Huntington's disease, and determine a second assessment of the one or more symptoms of Huntington's disease based on the extracted second plurality of features.

In some embodiments, the one or more symptoms of Huntington's disease in the subject include at least one of a symptom indicative of a cognitive function of the subject, a symptom indicative of a motor function of the subject, a symptom indicative of a behavioral function of the subject, or a symptom indicative of a functional capacity of the subject.

In some embodiments, the one or more symptoms of Huntington's disease in the subject are indicative of at least one of visuo-motor integration, visual attention, motor speed, cognitive processing speed, chorea, dystonia, visuo-motor coordination, fine motor impairment, upper-body or lower-body bradykinesia.

In some embodiments, the one or more sensors associated with the device comprise at least one of a first sensor disposed within the device or a second sensor worn by the subject and configured to communicate with the device.

In some embodiments, prompting the subject to perform the one or more diagnostic tasks includes at least one of prompting the subject to answer one or more questions or prompting the subject to perform one or more actions.

In some embodiments, the one or more diagnostic tasks are associated with at least one of a EQ-5D-5L test (https://euroqol.org/eq-5d-instruments/eq-5d-5l-about/), Work Productivity and Activity Impairment (WPAI)-HD test, HD-Strength Deployment Inventory (SDI) test, speed tapping test, draw a shape test, chorea test, balance test, u-turn test, Symbol Digit Modalities Test (SDMT), and word reading test.

According to one aspect, the disclosure relates to a computer-implemented method for assessing one or more symptoms of Huntington's disease (HD) in a subject. The method includes receiving a plurality of first sensor data via one or more sensors associated with a device, extracting, from the received first sensor data, a first plurality of features associated with the one or more symptoms of Huntington's disease in the subject, and determining a first assessment of the one or more symptoms of Huntington's disease based on the extracted first plurality of features.

In some embodiments, the computer-implemented method further includes prompting the subject to perform one or more diagnostic tasks; in response to the subject performing the one or more diagnostics tasks, receiving, a plurality of second sensor data via the one or more sensors; extracting, from the received second sensor data, a second plurality of features associated with one or more symptoms of Huntington's disease; and determining a second assessment of the one or more symptoms of Huntington's disease based on at least the extracted second sensor data.

In some embodiments, the one or more symptoms of Huntington's disease in the subject include at least one of a symptom indicative of a cognitive function of the subject, a symptom indicative of a motor function of the subject, a symptom indicative of a behavioral function of the subject, or a symptom indicative of a functional capacity of the subject.

In some embodiments, the one or more symptoms of Huntington's disease in the subject are indicative of at least one of visuo-motor integration, visual attention, motor speed, cognitive processing speed, chorea, dystonia, visuo-motor coordination, fine motor impairment, upper-body or lower-body bradykinesia.

In some embodiments, the one or more sensors associated with the device is at least one of a first sensor disposed within the device or a second sensor located on the subject and configured to communicate with the device.

In some embodiments, prompting the subject to perform the one or more diagnostic tasks includes at least one of prompting the subject answer one or more questions or prompting the subject to perform one or more actions.

In some embodiments, the one or more diagnostic tasks are associated with at least one of a EQ-5D-5L test, WPAI-HD test, HD-SDI test, speed tapping test, draw a shape test, chorea test, balance test, a u-turn test, SDMT test, and word reading test.

According to one aspect of the disclosure, a non-transitory machine readable storage medium includes machine-readable instructions for causing a processor to execute a method for assessing one or more symptoms of Huntington's disease in a subject that includes receiving a plurality of sensor data via one or more sensors associated with a device; extracting, from the received sensor data, a plurality of features associated with the one or more symptoms of Huntington's disease in a subject; and determining an assessment of the one or more symptoms of Huntington's disease based on the extracted plurality of features.

### BRIEF DESCRIPTION OF THE DRAWINGS

A more complete understanding of aspects described herein and the advantages thereof may be acquired by referring to the following description in consideration of the accompanying drawings, in which like reference numbers indicate like features, and wherein:
FIG. 1 is a diagram of an example environment in which a diagnostic device for assessing one or more symptoms of Huntington's disease in a subject is provided according to an example embodiment.
FIG. 2 is a flow diagram of a method for assessing one or more symptoms of Huntington's disease in a subject based on passive monitoring of the subject according to an example embodiment.
FIG. 3 is a flow diagram of a method for assessing one or more symptoms of Huntington's disease in a subject based on active testing of the subject according to an example embodiment.
FIGS. 4A-4B depict example screenshots illustrating the pull-down menus of an example diagnostic application according to one or more illustrative aspects described herein.
FIGS. 5A-5D depict example screenshots illustrating a selection of the menu options of the first pull-down menu shown in FIG. 4A according to one or more illustrative aspects described herein.
FIGS. 6A-6D depict example screenshots illustrating a selection of various menu options of the second pull-down menu shown in FIG. 4B according to one or more illustrative aspects described herein.
FIGS. 7A-7C depict example screenshots illustrating a selection of the "Settings" menu option of the second pull-down menu shown in FIG. 4B according to one or more illustrative aspects described herein.
FIGS. 8A-8G depict example screenshots illustrating a selection of the "Initialize" menu option of the second pull-down menu shown in FIG. 4B according to one or more illustrative aspects described herein.
FIGS. 9A-9F depict example screenshots illustrating a selection of the "Begin Full Active Tests" menu option of the second pull-down menu shown in FIG. 4B according to one or more illustrative aspects described herein.
FIGS. 10A and 10B depict example screenshots illustrating example daily questions according to one or more illustrative aspects described herein.
FIGS. 11A-11I depict example screenshots illustrating an example EQ-5D-5L test according to one or more illustrative aspects described herein.
FIGS. 12A-12J depict example screenshots illustrating an example WPAI-HD according to one or more illustrative aspects described herein.
FIG. 13 depicts an example screenshot 1305 illustrating an example HD-SDI test according to one or more illustrative aspects described herein.
FIGS. 14A-14J depict example screenshots illustrating an example speeded tapping test according to one or more illustrative aspects described herein.
FIGS. 15A-15E depict example screenshots from an instructional video for an example speeded tapping test according to one or more illustrative aspects described herein.
FIGS. 16A-16T depict example screenshots illustrating an example draw a shape test according to one or more illustrative aspects described herein.
FIGS. 17A-17E depict example screenshots from an example instructional video for a draw a shape test according to one or more illustrative aspects described herein.
FIGS. 18A-18J depict example screenshots illustrating an example chorea test according to one or more illustrative aspects described herein.
19A-19E depict example screenshots from an example instructional video for a chorea test according to one or more illustrative aspects described herein.
FIGS. 20A-20H depict example screenshots illustrating an example balance test according to one or more illustrative aspects described herein.
FIGS. 21A-21D depict example screenshots from an instructional video for an example balance test according to one or more illustrative aspects described herein.
FIGS. 22A-22H depict example screenshots illustrating an example u-turn test according to one or more illustrative aspects described herein.
23A-23F depict example screenshots from an instructional video for an example u-turn test according to one or more illustrative aspects described herein.
FIGS. 24A-24G depict example screenshots illustrating an example walk test according to one or more illustrative aspects described herein.
FIGS. 25A-25D depict example screenshots illustrating an example SDMT test according to one or more illustrative aspects described herein.
FIGS. 26A-26D depict example screenshots illustrating an example word reading test according to one or more illustrative aspects described herein.
FIGS. 27A-27B depict example screenshots of the instructional video for a word reading test according to one or more illustrative aspects described herein.
FIGS. 28A-28Q depict example screenshots illustrating the various messages and/or warnings displayed by the diagnostic application according to one or more illustrative aspects described herein.
FIG. 29 is a table showing various characteristics of the group of patients participating in a study.
FIG. 30 is a graph showing the number of patients completing active tests per week for the first 20 weeks after the in-clinic screening visit.
FIG. 31 is a graph showing the number of patients completing active tests per week for the first 20 weeks after the in-clinic screening visit.
FIG. 32 is a plot showing a correlation between results measured using a smartphone application's SDMT active test and results from standard in-clinic SDMT.
FIG. 33 is a plot showing a correlation between results measured using the smartphone application's Stroop word reading active test and results from standard in-clinic Stroop word reading testing.
FIG. 34 is a plot showing a correlation between results measured using the smartphone application's speeded tapping active test and results from standard in-clinic speeded tapping testing.
FIG. 35 is a plot showing a correlation between results measured using the smartphone application's chorea test and results from standard in-clinic chorea assessment.
FIG. 36 illustrates one example of a network architecture and data processing device that may be used to implement one or more illustrative aspects described herein.

### DETAILED DESCRIPTION

In the following description of various aspects, reference is made to the accompanying drawings, which form a part hereof, and in which is shown by way of illustration various embodiments in which aspects described herein may be practiced. It is to be understood that other aspects and/or embodiments may be utilized and structural and functional modifications may be made without departing from the scope of the described aspects and embodiments. Aspects described herein are capable of other embodiments and of being practiced or being carried out in various ways. Also, it is to be understood that the phraseology and terminology used herein are for the purpose of description and should not be regarded as limiting. Rather, the phrases and terms used herein are to be given their broadest interpretation and meaning. The use of "including" and "comprising" and variations thereof is meant to encompass the items listed thereafter and equivalents thereof as well as additional items and equivalents thereof. The use of the terms "mounted," "connected," "coupled," "positioned," "engaged" and similar terms, is meant to include both direct and indirect mounting, connecting, coupling, positioning and engaging.

Systems, methods and devices described herein provide a diagnostic for assessing one or more symptoms of Huntington's disease in a patient. In some embodiments, the diagnostic may be provided to the patient as a software application installed on a mobile device.

In some embodiments, systems, methods and devices described herein provide a diagnostic for assessing one or more symptoms of Huntington's disease in a patient based on passive monitoring of the patient. In some embodiments, the diagnostic obtains or receives sensor data from one or more sensors associated with the mobile device as the patient performs activities of daily life. In some embodiments, the sensors may be within the mobile device or wearable sensors. In some embodiments, the sensor features associated with the symptoms of Huntington's disease are extracted from the received or obtained sensor data. In some embodiments, the assessment of the symptom severity and progression of Huntington's disease in the patient is determined based on the extracted sensor features.

In some embodiments, systems, methods and devices according to the present disclosure provide a diagnostic for assessing one or more symptoms of Huntington's disease in a patient based on active testing of the patient. In some embodiments, the diagnostic prompts the patient to perform diagnostic tasks. In some embodiments, the diagnostic tasks are anchored in or modelled after established methods and standardized tests; in others, new tests or methods may be used. In some embodiments, in response to the patient performing the diagnostic task, the diagnostic obtains or receives sensor data via one or more sensors. In some embodiments, the sensors may be within a mobile device or wearable sensors worn by the patient. In some embodiments, sensor features associated with the symptoms of Huntington's disease are extracted from the received or obtained sensor data. In some embodiments, the assessment of the symptom severity and progression of Huntington's disease in the patient is determined based on the extracted features of the sensor data.

Assessments of symptom severity and progression of Huntington's disease using diagnostics according to the present disclosure correlate sufficiently with the assessments based on clinical results and may thus replace clinical patient monitoring and testing. Example diagnostics according to the present disclosure may be used in an out of clinic environment, and therefore have advantages in cost, ease of patient monitoring and convenience to the patient. This facilitates frequent patient monitoring and testing, resulting in a better understanding of the disease stage and provides insights about the disease that are useful to both the clinical and research community. An example diagnostic according to the present disclosure can provide earlier detection of even small changes in symptoms of Huntington's disease in a patient and can therefore be used for better disease management including individualized therapy.

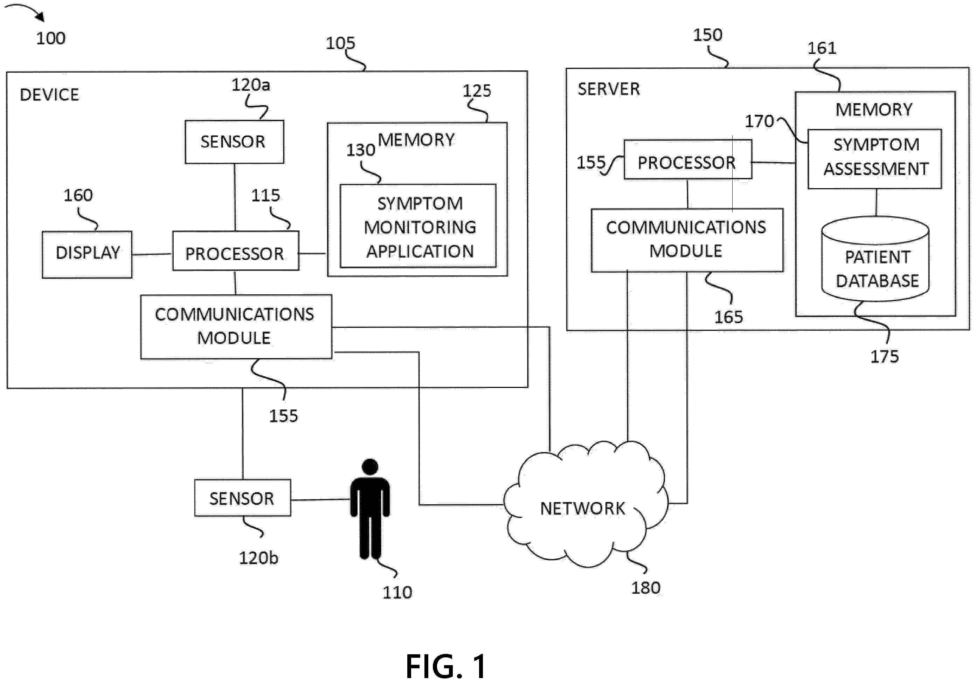

FIG. 1 is a diagram of an example environment 100 in which a diagnostic device 105 for assessing one or more symptoms of Huntington's disease in a subject 110 is provided. In some embodiments, the device 105 may be a smartphone, a smartwatch or other mobile computing device. The device 105 includes a display screen 160. In some embodiments, the display screen 160 may be a touchscreen. The device 105 includes at least one processor 115 and a memory 125 storing computer-instructions for a symptom monitoring application 130 that, when executed by the at least one processor 115, cause the device 105 to assess the one or more symptoms of Huntington's disease in the subject 110 based on passive monitoring of the subject 110. The device 105 receives a plurality of sensor data via one or more sensors associated with the device 105. In some embodiments, the one or more sensors associated with the device is at least one of a sensor disposed within the device or a sensor worn by the subject and configured to communicate with the device. In FIG. 1, the sensors associated with the device 105 include a first sensor 120a that is disposed within the device 105 and a second sensor 120b that is worn by the subject 110. The device 105 receives a plurality of first sensor data via the first sensor 120a and a plurality of second sensor data via the second sensor 120b as the subject 110 performs activities of daily life.

The device 105 extracts, from the received first sensor data and second sensor data, features associated with one or more symptoms of Huntington's disease in the subject 110. In some embodiments, the symptoms of Huntington's disease in the subject 110 may include a symptom indicative of a cognitive function of the subject 110, a symptom indicative of a motor function of the subject 110, a symptom indicative of a behavioral function of the subject 110, or a symptom indicative of a functional capacity of the subject 110. In some embodiments, the one or more symptoms of Huntington's disease in the subject 110 are indicative of at least one of visuo-motor integration, visual attention, motor speed, cognitive processing speed, chorea, dystonia, visuo-motor coordination, fine motor impairment, upper-body or lower-body bradykinesia.

In some embodiments, the first sensor 120a or second sensor 120b (or another sensor altogether) associated with the device 105 may include or interface with a satellite-based radio navigation system, such as may be used with the Global Positioning System (GPS), Galileo, GLONASS, and/or similar systems (collectively referred to herein as GPS), and the plurality of first sensor data received from the first sensor 120b may include location data associated with the device 105. In some embodiments, the device 105 extracts location data, from the received first sensor data and second sensor data, associated with one or more symptoms of Huntington's disease in the subject 110. In some embodiments, an assessment of motor function of the subject 110 may be based at least in part on the extracted location data (e.g., patient mobility may be assessed based in part on GPS location data). In some embodiments, the sensors 120 associated with the device 105 may include sensors associated with Bluetooth and WiFi functionality and the sensor data may include information associated with the Bluetooth and WiFi signals received by the sensors 120. In some embodiments, the device 105 extracts data corresponding to the density of Bluetooth and WiFi signals received or transmitted by the device 105 or sensors, from the received first sensor data and second sensor data. In some embodiments, an assessment of behavioral function or an assessment of the functional capacity of the subject 110 may be based on the extracted Bluetooth and WiFi signal data (e.g., an assessment of patient sociability may be based in part on the density of Bluetooth and WiFi signals picked up).

The device 105 determines an assessment of the one or more symptoms of Huntington's disease in the subject 110 based on the extracted features of the received first and second sensor data. In some embodiments, the device 105 send the extracted features over a network 180 to a server 150. The server 150 includes at least one processor 155 and a memory 161 storing computer-instructions for a symptom assessment application 170 that, when executed by the server processor 155, cause the processor 155 to determine an assessment of the one or more symptoms of Huntington's disease in the subject 110 based on the extracted features received by the server 150 from the device 105. In some embodiments, the symptom assessment application 170 may determine an assessment of the one or more symptoms of Huntington's disease in the subject 110 based on the extracted features of the sensor data received from the device 105 and a patient database 175 stored in the memory 160. In some embodiments, the patient database 175 may include patient and/or clinical data. In some embodiments, the patient database 175 may include in-clinic and sensor-based measures of motor and cognitive function at baseline and longitudinal from early Huntington's disease patients. In some embodiments, the patient database 175 may include in-clinic and sensor-based measures of behavioral and other symptoms. In some embodiments, the patient database 175 may include data from patients at other stages of Huntington's disease. In some embodiments, the patient database 175 may be independent of the server 150. In some embodiments, the server 150 sends the determined assessment of the one or more symptoms of Huntington's disease in the subject 110 to the device 105. In some embodiments, the device 105 may output the assessment of the one or more symptoms of Huntington's disease. In some embodiments, the device 105 may communicate information to the subject 110 based on the assessment. In some embodiments, the assessment of the one or more symptoms of Huntington's disease may be communicated to a clinician that may determine individualized therapy for the subject 110 based on the assessment.

In some embodiments, the computer-instructions for the symptom monitoring application 130, when executed by the at least one processor 115, cause the device 105 to assess one or more symptoms of Huntington's disease in the subject 110 based on active testing of the subject 110. The device 105 prompts the subject 110 to perform one or more diagnostic tasks. In some embodiments, prompting the subject to perform the one or more diagnostic tasks includes prompting the subject to answer one or more questions or prompting the subject to perform one or more actions. In some embodiments, the diagnostic tasks are anchored in or modelled after well-established methods and standardized tests for evaluating and assessing Huntington's disease.

In response to the subject 110 performing the one or more diagnostic tasks, the diagnostic device 105 receives a plurality of sensor data via the one or more sensors associated with the device 105. As mentioned above, the sensors associated with the device 105 may include a first sensor 120a that is disposed within the device 105 and a second sensor 120b that is worn by the subject 110. The device 105 receives a plurality of first sensor data via the first sensor 120a and a plurality of second sensor data via the second sensor 120b. In some embodiments, the one or more diagnostic tasks may be associated with at least one of a EQ-5D-5L test, a WPAI-HD test, HD-SDI test, a speed tapping test, a draw a shape test using a left hand of the subject, a draw a shape test using a right hand of the subject, a chorea test, a balance test, a u-turn test, a SDMT test, and/or a word reading test.

The device 105 extracts, from the received plurality of first sensor data and the received plurality of second sensor data, features associated with one or more symptoms of Huntington's disease in the subject 110. The symptoms of Huntington's disease in the subject 110 may include a symptom indicative of a cognitive function of the subject 110, a symptom indicative of a motor function of the subject 110, a symptom indicative of a behavioral function of the subject 110, or a symptom indicative of a functional capacity of the subject 110. In some embodiments, the one or more symptoms of Huntington's disease in the subject 110 are indicative of at least one of visuo-motor integration, visual attention, motor speed, cognitive processing speed, chorea, dystonia, visuo-motor coordination, fine motor impairment, upper-body or lower-body bradykinesia. As discussed above, location-based data from a GPS or similar system may be used to assess symptoms related to the motor function and/or mobility of the subject and other location based assessments. Similarly as discussed above, WiFi and Bluetooth signal density may be used, e.g., to help assess patent sociability.

The device 105 determines an assessment of the one or more symptoms of Huntington's disease in the subject 110 based on the extracted features of the received first and second sensor data. In some embodiments, the device 105 sends the extracted features over a network 180 to a server 150. The server 150 may include at least one processor 155 and a memory 161 storing computer-instructions for a symptom assessment application 170 that, when executed by the server processor 155, cause the processor 155 to determine an assessment of the one or more symptoms of Huntington's disease in the subject 110 based on the extracted features received by the server 150 from the device 105. In some embodiments, the symptom assessment application 170 may determine an assessment of the one or more symptoms of Huntington's disease in the subject 110 based on the extracted features of the sensor data received from the device 105 and a patient database 175 stored in the memory 160. In some embodiments, the patient database 175 may include patient and/or clinical data. In some embodiments, the patient database 175 may include in-clinic and sensor-based measures of motor and cognitive function at baseline and longitudinal from early Huntington's disease patients. In some embodiments, the patient database 175 may include in-clinic and sensor-based measures of behavioral and other symptoms. In some embodiments, the patient database 175 may include data from patients at other stages of Huntington's disease. In some embodiments, the patient database 175 may be independent of the server 150. In some embodiments, the server 150 sends the determined assessment of the one or more symptoms of Huntington's disease in the subject 110 to the device 105. In some embodiments, the device 105 may output the assessment of the one or more symptoms of Huntington's disease. In some embodiments, the device 105 may communicate information to the subject 110 based on the assessment. In some embodiments, the assessment of the one or more symptoms of Huntington's disease may be communicated to a clinician that may determine individualized therapy for the subject 110 based on the assessment.

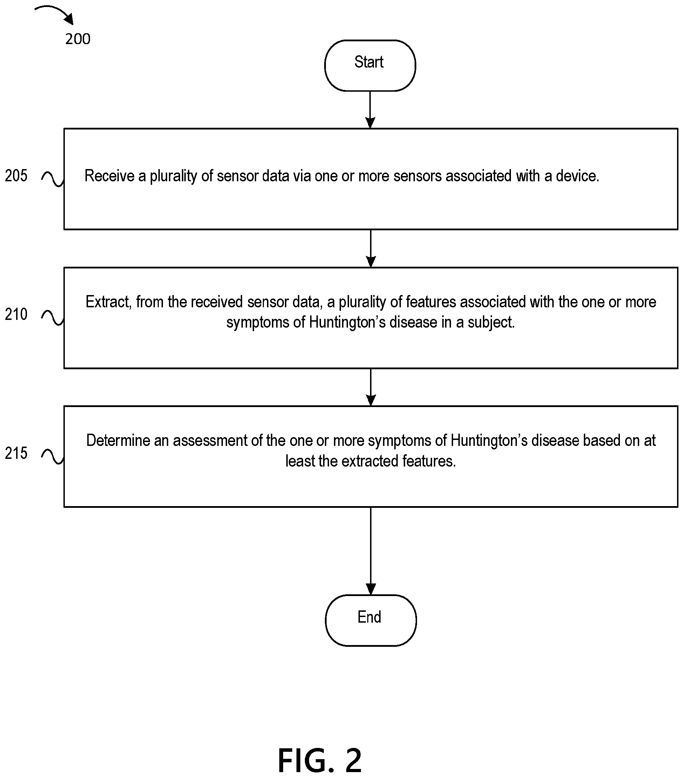

FIG. 2 illustrates an example method 200 for assessing one or more symptoms of Huntington's disease in a subject based on passive monitoring of the subject using the example device 105 of FIG. 1. While FIG. 2 is described with reference to FIG. 1, it should be noted that the method steps of FIG. 2 may be performed by other systems. The method 200 for assessing one or more symptoms of Huntington's disease in a subject includes receiving a plurality of sensor data via one or more sensors associated with a device (step 205). The method 200 includes extracting, from the received plurality of first sensor data, a plurality of features associated with the one or more symptoms of Huntington's disease in the subject (step 210). The method 200 also includes determining a first assessment of the one or more symptoms of Huntington's disease based on the extracted features (step 215).

FIG. 2 sets forth an example method 200 for assessing one or more symptoms of Huntington's disease using the example device 105 in FIG. 1. In some embodiments, the device 105 may be a smartphone, a smartwatch or other mobile computing device. The device 105 includes at least one processor 115 and a memory 125 storing computer-instructions for a symptom monitoring application 130 that, when executed by the at least one processor 115, cause the device 105 to assess the one or more symptoms of Huntington's disease in the subject 110 based on passive monitoring of the subject 110.

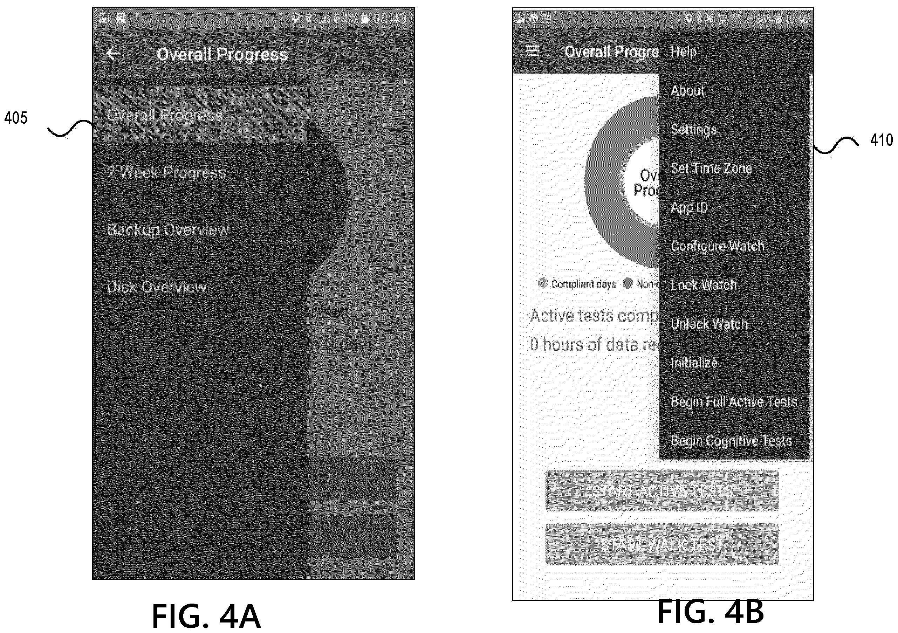

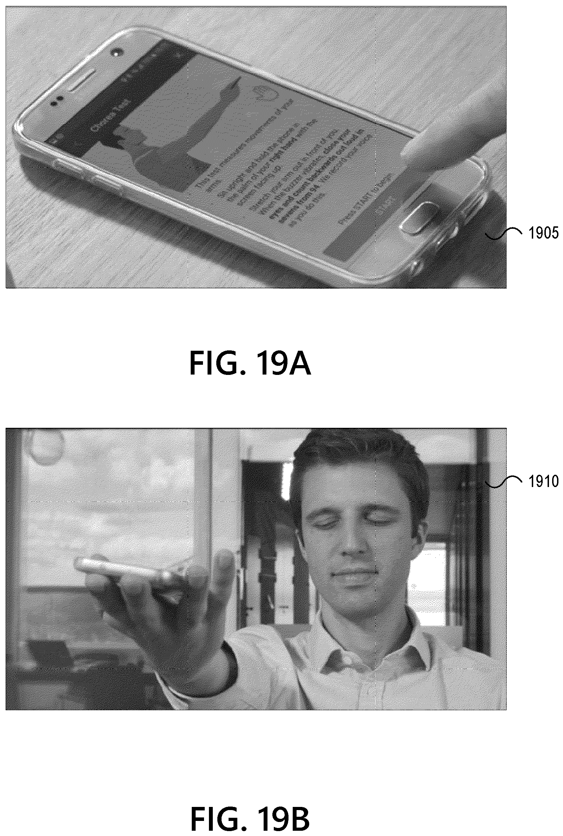

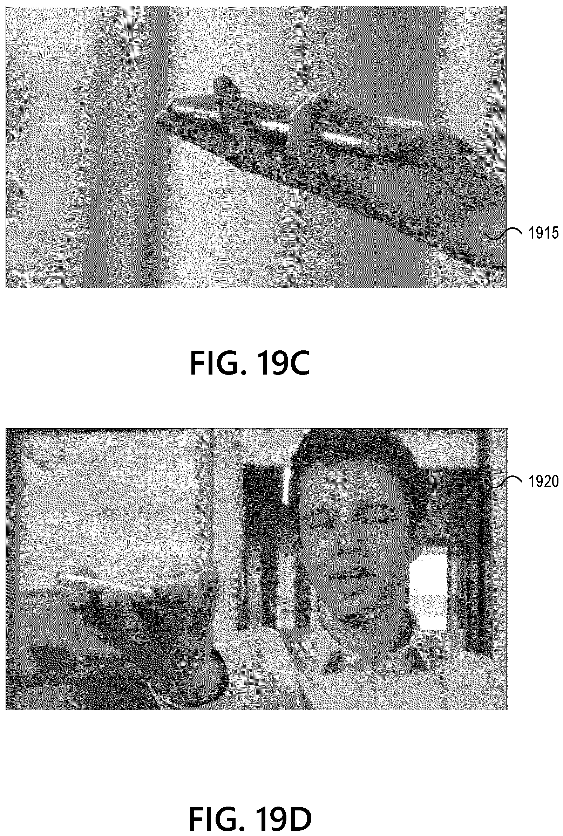

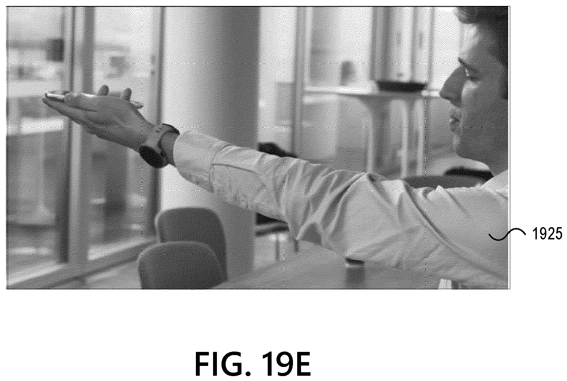

In some embodiments, the symptom monitoring application 130 may provide a diagnostic application that includes a user interface (UI) that is displayed on the display screen 160 of the device 105. In some embodiments, the display screen 160 may be a touchscreen and the user interacts with the diagnostic application via the displayed UI. FIGS. 4-28 depict example screenshots, illustrating the UI of an example diagnostic application according to illustrative aspects described herein, and responsive UI changes to the user interface as a user interacts with the diagnostic application.

FIGS. 4A-4B depict example screenshots 405 and 410 illustrating pull-down menus of an example diagnostic application according to one or more illustrative aspects described herein. The screenshot 405 of FIG. 4A shows a first pull-down menu with the menu options "Overall Progress," "2 Week Progress," "Backup Overview," and "Disk Overview," which a user can select, respectively, to instruct the diagnostic application to perform the requested menu action, and as further described below. FIG. 4B is a screenshot 410, showing a second pull-down menu 410 with the menu options "Help," "About," "Settings," "Set Time Zone," "App ID," "Configure Watch," "Lock Watch," "Unlock Watch," "Initialize," "Begin Full Active Tests," and "Begin Cognitive Tests." Similarly, when user selects one of the menu items, the diagnostic application performs the requested task or provides the requested information, and as further described below.

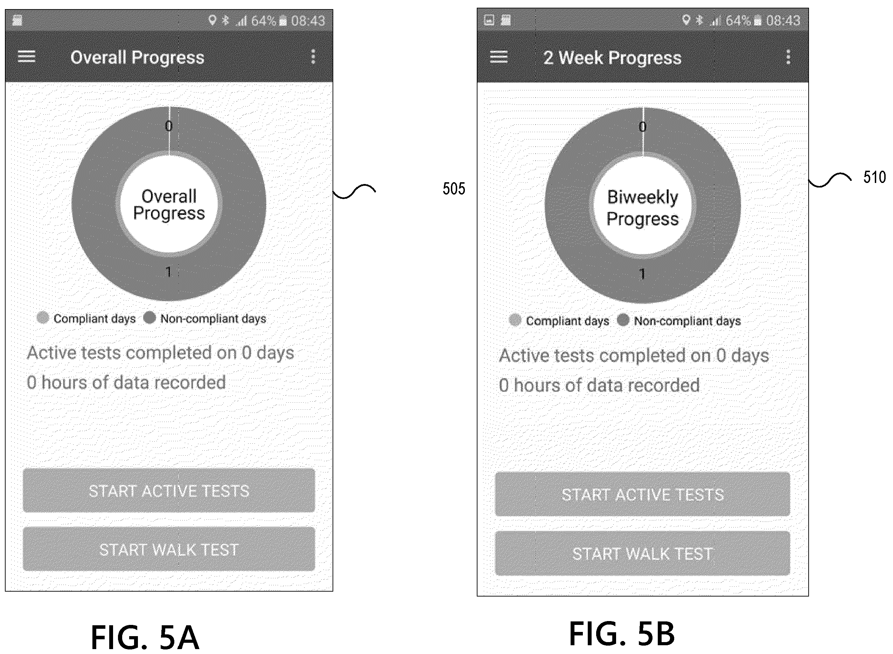

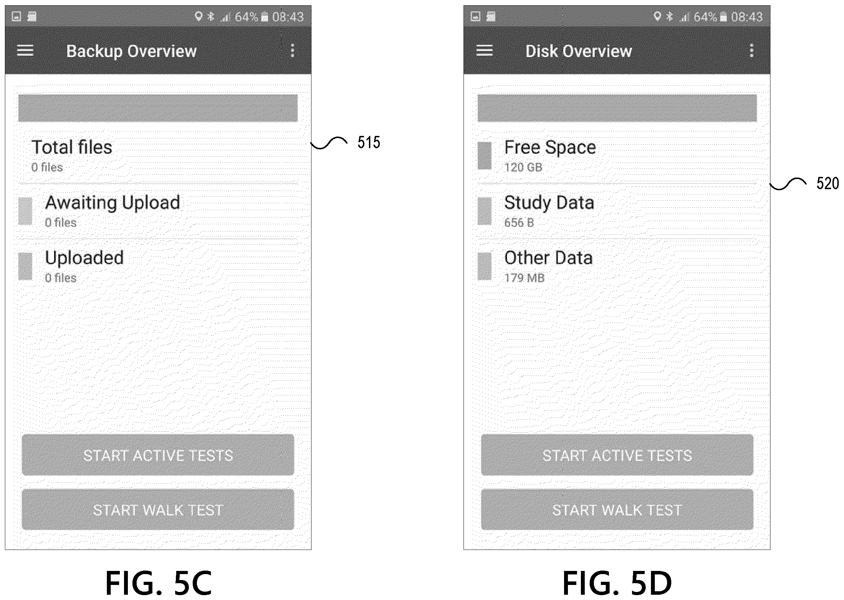

FIGS. 5A-5D depict example screenshots 505, 510, 515, and 520 illustrating a selection of the menu options of the first pull-down menu shown in FIG. 4A according to according to one or more illustrative aspects described herein. As shown in the screenshot 505 of FIG. 5A, selecting the "Overall Progress" menu option displays information about the overall progress of the subject. As shown in the screenshot 510 of FIG. 5B, selecting the "2 Week Progress" menu option displays information 510 about the biweekly progress of the subject. As shown in the screenshot 515 of FIG. 5C, selecting the "Backup Overview" menu option displays information about the uploading of files, for example, the number of files that are awaiting to be uploaded or the number of files that have already been uploaded. As shown in the screenshot 520 of FIG. 5D, selecting the "Disk Overview" menu option displays information about the memory usage of the device 105 associated with data of the diagnostic application.

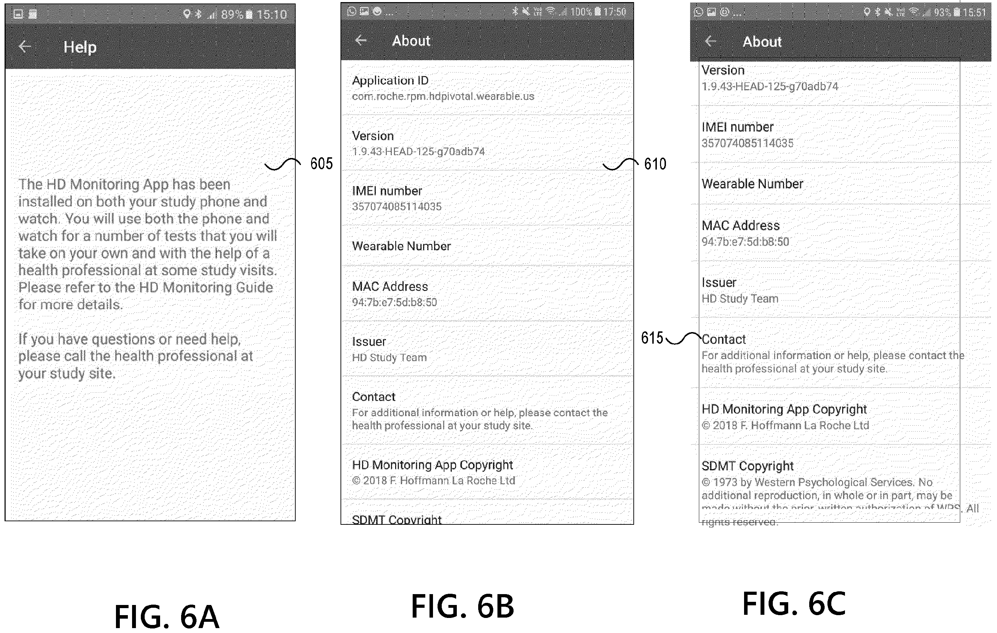

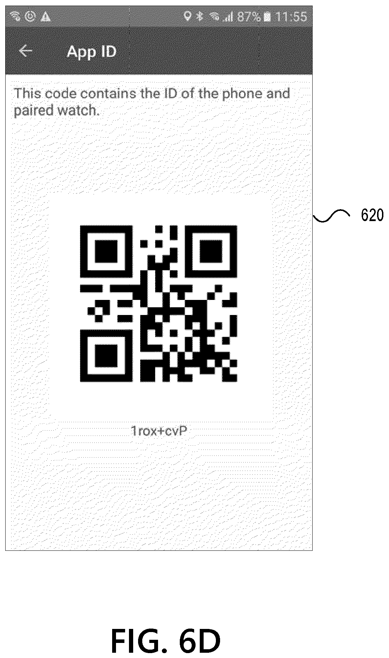

FIGS. 6A-6D depict example screenshots 605, 610, 615, and 620 illustrating a selection of various menu options of the second pull-down menu shown in FIG. 4B according to one or more illustrative aspects described herein. As shown in the screenshot 605 of FIG. 6A, selecting the "Help" menu option displays general information about the diagnostic application. As shown in the screenshot 610 of FIG. 6B, selecting the "About" menu option may display information including information about the application such as the Application ID, Version, Contact information, and various Copyright information. Figure 6C is an example screenshot 615 showing additional information displayed by scrolling down past the information shown in the screenshot 610 of FIG. 6B. In some embodiments, the device 105 may be paired with a second device, such as a smartwatch. As shown in the screenshot 620 of FIG. 6D, selecting the "App ID" menu option displays information about the pairing between the device 105 and the second device. In some embodiments, the second device may be one or more wearable sensors, such as the second sensor 120b shown in FIG. 1. In some embodiments, the second device may be any device that includes a motion sensor with an inertial measurement unit (IMU). In some embodiments, the second device may be a group of devices or sensors. The second device may comprise or be incorporated into a wearable such as a smart watch, electronic health monitor, and the like.

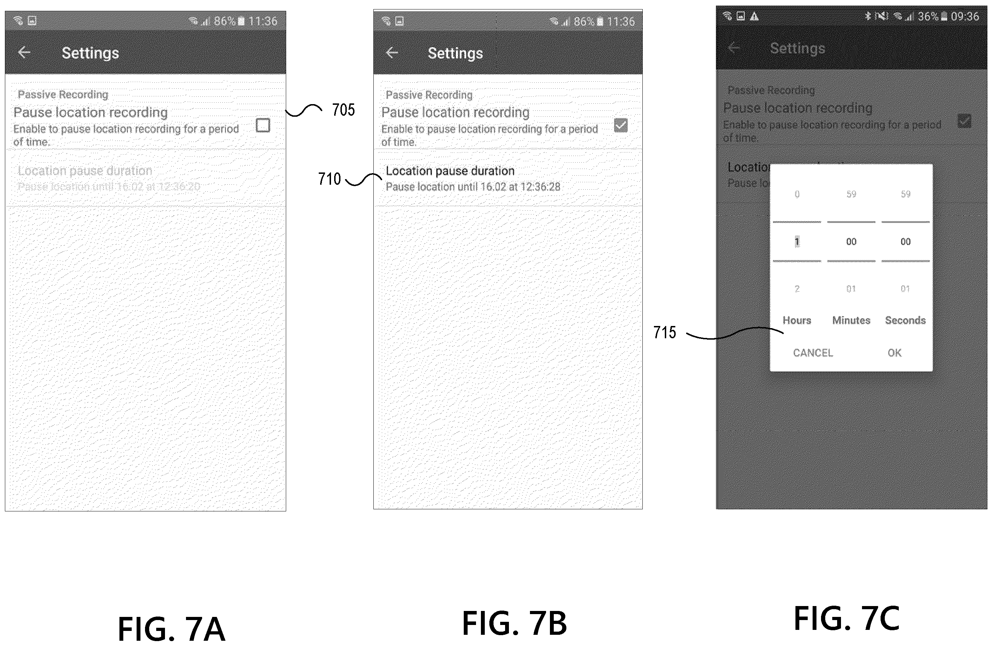

In some embodiments, the user may specify settings for passive monitoring of the user by the device 105 by selecting the "Settings" menu option of the second pull-down menu shown in FIG. 4B. FIGS. 7A-7C depict example screenshots 705, 710 and 715 illustrating a selection of the "Settings" menu option of the second pull-down menu shown in FIG. 4B according to one or more illustrative aspects described herein. As shown in the screenshots 705 and 710 of FIGS. 7A and 7B, selecting the "Settings" menu option displays various settings for "Passive Recording," such as "Pause location recording" and "Location pause duration". Selecting "Pause location recording" enables the user to pause location recording for a period of time. This pauses passive monitoring of the subject 110 by the device 105 for a period of time specified by "Location pause duration". The hour, minute and seconds of the period of time may be specified via a pop-up, as shown in the screenshot 715 of FIG. 7C. When the "Pause location recording" is disabled or not enabled, the device 105 receives a plurality of first sensor data via the first sensor 120a and a plurality of second sensor data via the second sensor 120b.

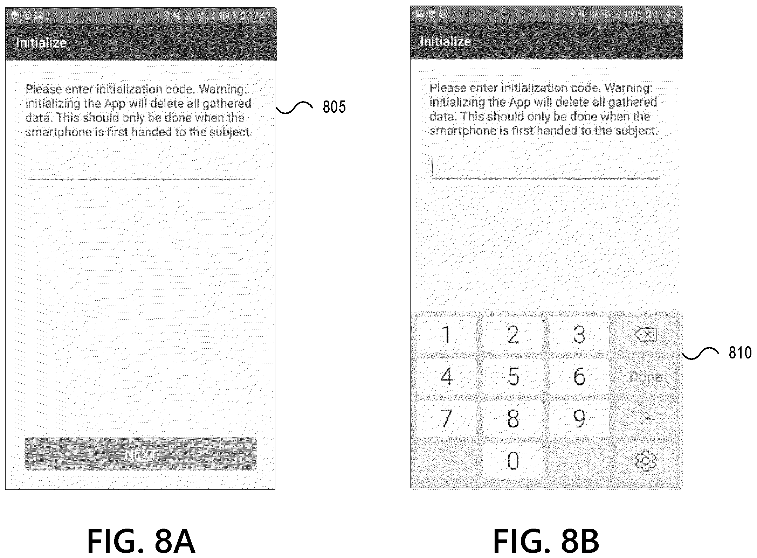

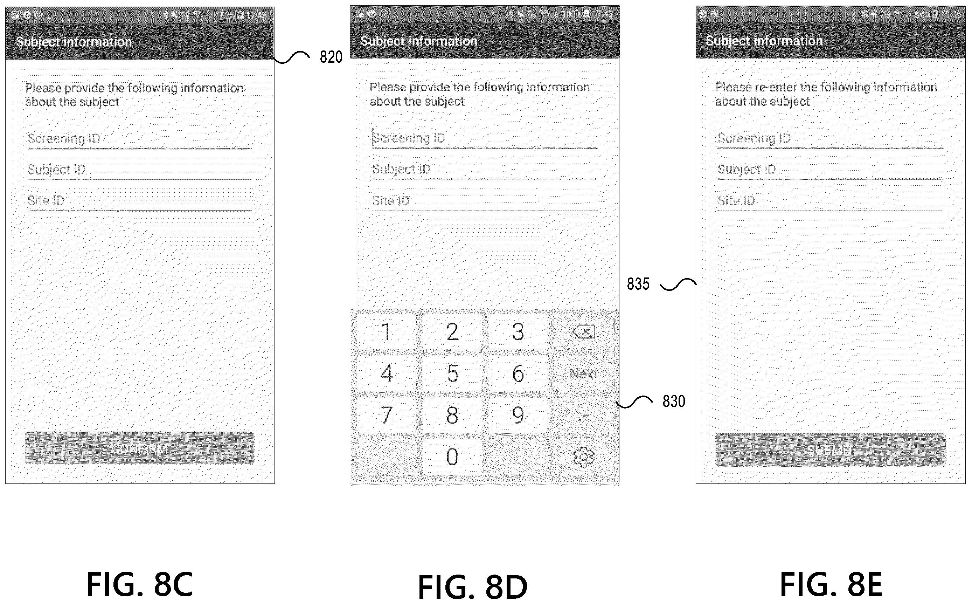

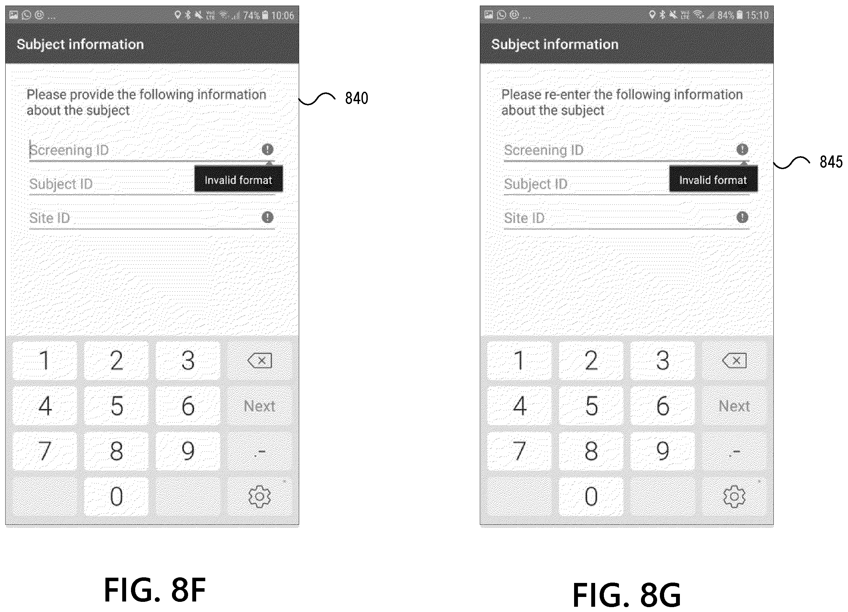

FIGS. 8A-8G depict example screenshots 805, 810, 815, 820, 825, 830, 835, 840 and 845, illustrating a selection of the "Initialize" menu option of the second pull-down menu shown in the screenshot 410 of FIG. 4B according to one or more illustrative aspects described herein. As shown in the screenshot 805 FIG. 8A, selecting the "Initialize" menu option of the second pull-down menu shown in the screenshot 410 of FIG. 4B displays a request for information for initializing the device 105. In some embodiments, the requested information may be an initialization code. In some embodiments, the initialization of the device 105 is performed when the device 105 is initially given to the subject 110. As shown in the screenshot 810 of FIG. 8B, a keypad may be displayed in order to enter the requested initialization information. Additionally, as shown in the screenshot 815 of FIG. 8C, information about the subject 110 may also be provided. As shown in the screenshot 815 of FIG. 8C, the requested subject information may include a "Screening ID," a "Subject ID," and a "Site ID." As shown in the screenshot 820 of FIG. 8D, a pop-up keypad may be displayed. FIG. 8E depicts an example screenshot 835 requesting re-entry of the subject information if it is incorrect. As shown in the example screenshots 840 and 845 in FIGS. 8F and 8G, if the subject information is requested, invalid formatting of the subject information is flagged for re-entry.

Referring back to FIG. 2, the method 200 begins by proceeding to step 205 which includes receiving a plurality of sensor data via one or more sensors associated with the device 105. During passive monitoring of the subject 110, the device 105 receives a plurality of sensor data via one or more sensors associated with the device 105. The sensors associated with the device 105 include a first sensor 120a that is disposed within the device 105 and a second sensor 120b that is worn by the subject 110. The device 105 receives a plurality of first sensor data via the first sensor 120a and a plurality of second sensor data via the second sensor 120b.

Referring back to FIGS. 7A-7C, various aspects of passive monitoring may be specified via the user interface of the symptom monitoring application 130. As shown in the example screenshots 705 and 710 of FIGS. 7A and 7B, selecting the "Settings" menu option displays settings associated with "Passive Recording," such as "Pause location recording" and "Location pause duration." Selecting "Pause location recording" 705 enables the user to pause location recording for a period of time. This pauses passive monitoring of the subject 110 by the device 105 for a period of time specified by "Location pause duration." The hour, minute and seconds of the period of time may be specified via a pop-up, as shown in the screenshot 715 of FIG. 7C. When the "Pause location recording" is disabled or not enabled, the device 105 receives a plurality of first sensor data via the first sensor 120a and a plurality of second sensor data via the second sensor 120b.

The method 200 proceeds to step 210 which includes extracting, from the received first sensor data, a first plurality of features associated with the one or more symptoms of Huntington's disease in a subject. The device 105 extracts, from the received first sensor data and second sensor data, features associated with one or more symptoms of Huntington's disease in the subject 110. The symptoms of Huntington's disease in the subject 110 may include a symptom indicative of a cognitive function of the subject 110, a symptom indicative of a motor function of the subject 110, a symptom indicative of a behavioral function of the subject 110, or a symptom indicative of a functional capacity of the subject 110. In some embodiments, the extracted features of the plurality of first and second sensor data may be indicative of symptoms of Huntington's disease such as visuo-motor integration, visual attention, motor speed, cognitive processing speed, chorea, dystonia, visuo-motor coordination, fine motor impairment, upper-body or lower-body bradykinesia.

The method 200 proceeds to step 215 which includes determining an assessment of the one or more symptoms of Huntington's disease based on the extracted features. The device 105 determines an assessment of the one or more symptoms of Huntington's disease in the subject 110 based on the extracted features of the received first and second sensor data. In some embodiments, the device 105 may send the extracted features over a network 180 to a server 150. The server 150 includes at least one processor 155 and a memory 160 storing computer-instructions for a symptom assessment application 170 that, when executed by the processor 155, determine an assessment of the one or more symptoms of Huntington's disease in the subject 110 based on the extracted features received by the server 150 from the device 105. In some embodiments, the symptom assessment application 170 may determine an assessment of the one or more symptoms of Huntington's disease in the subject 110 based on the extracted features of sensor data received from the device 105 and a patient database 175 stored in the memory 160. The patient database 175 may include various clinical and/or patient data. In some embodiments, the patient database 175 may include data, such as, in-clinic and sensor-based measures of motor and cognitive function at baseline and longitudinal from early Huntington's disease patients. In some embodiments, the patient database 175 may include in-clinic and sensor-based measures of behavioral and other symptoms. In some embodiments, the patient database 175 may include data of patients at other stages of Huntington's disease. In some embodiments, the patient database 175 may be independent of the server 150. In some embodiments, the server 150 sends the determined assessment of the one or more symptoms of Huntington's disease in the subject 110 to the device 105. In some embodiments, the device 105 may output the assessment of the one or more symptoms of Huntington's disease on the display 160 of the device 105. In some embodiments, the assessment of the one or more symptoms of Huntington's disease may be communicated to a clinician that may determine individualized therapy for the subject 110 based on the assessment.

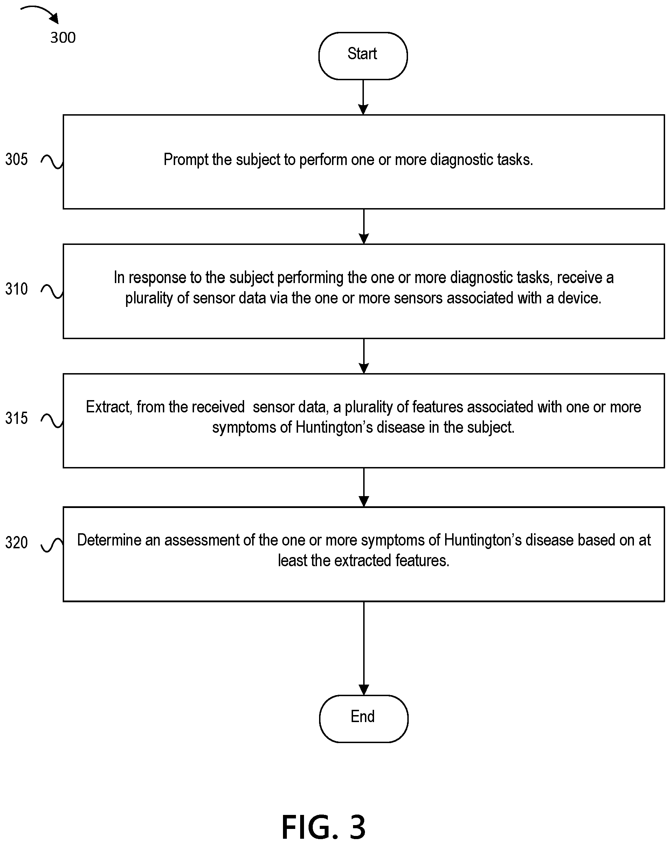

FIG. 3 illustrates an example method 300 for assessing one or more symptoms of Huntington's disease in a subject based on active testing of the subject using the example device 105 of FIG. 1. While FIG. 3 is described with reference to FIG. 1, it should be noted that the method steps of FIG. 3 may be performed by other systems. The method 300 includes prompting the subject to perform one or more diagnostic tasks (305). The method 300 includes receiving, in response to the subject performing the one or more tasks, a plurality of sensor data via the one or more sensors (step 310). The method 300 includes extracting, from the received sensor data, a plurality of features associated with one or more symptoms of Huntington's disease (315). The method 300 includes determining an assessment of the one or more symptoms of Huntington's disease based on at least the extracted sensor data (step 320).

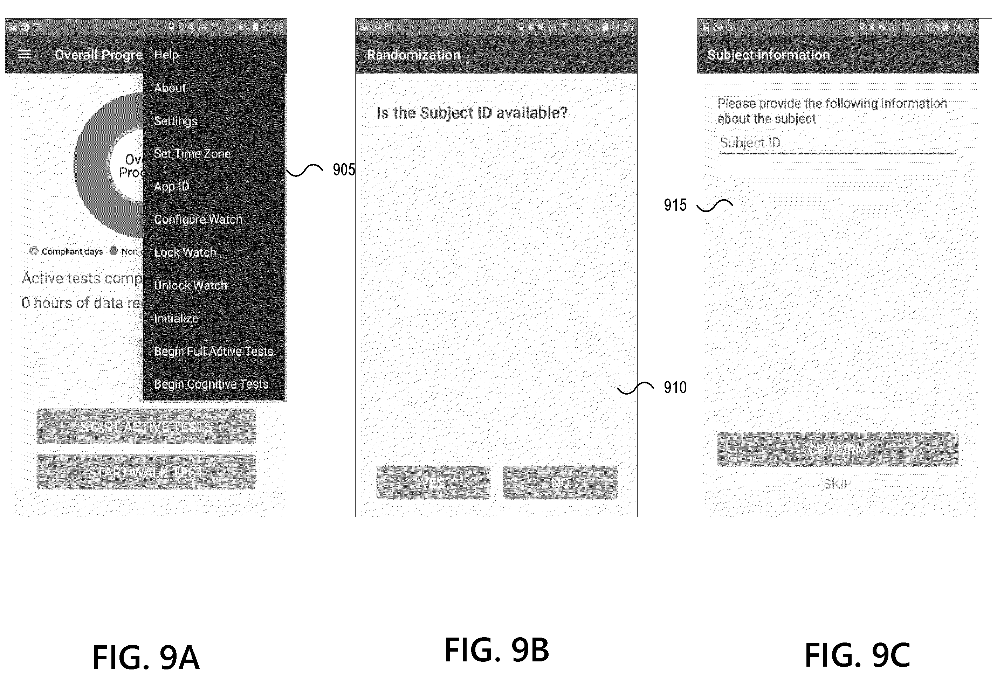

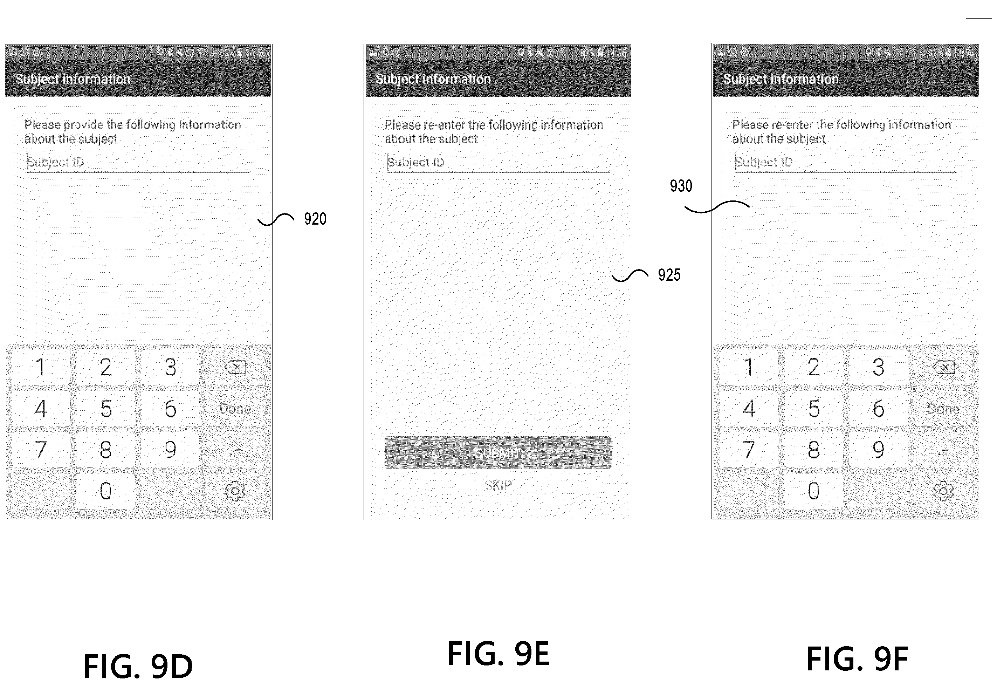

FIG. 3 sets forth an example method 300 for assessing one or more symptoms of Huntington's disease based on active testing of the subject 110 using the example device 105 in FIG. 1. In some embodiments, active testing of the subject 110 using the device 105 may be selected via the user interface of the symptom monitoring application 130. FIGS. 9A-9F depict example screenshots 905, 910, 915, 920, 925 and 930 illustrating a selection of the "Begin Full Active Tests" menu option of the second pull-down menu 410 shown in FIG. 4B according to one or more illustrative aspects described herein. FIG. 9A is an example screenshot 905 of the second pull-down menu also shown in the screenshot 410 FIG. 4B. In some embodiments, upon selecting the "Begin Full Active Tests" menu option, the user interface may display a request for the Subject ID as shown in the example screenshots 910, 915, 920, 925, and 930 of FIGS. 9B-9F. Upon entering the requested Subject ID information, the device 105 begins to perform active testing of the subject 110.

The method 300 begins by proceeding to step 305 which includes prompting the subject to perform one or more diagnostic tasks. The device 105 prompts the subject 110 to perform one or more diagnostic tasks. In some embodiments, prompting the subject to perform the one or more diagnostic tasks includes prompting the subject to answer one or more questions or prompting the subject to perform one or more actions. In some embodiments, the diagnostic tasks are anchored in or modelled after well-established methods and standardized tests for evaluating and assessing Huntington's disease.

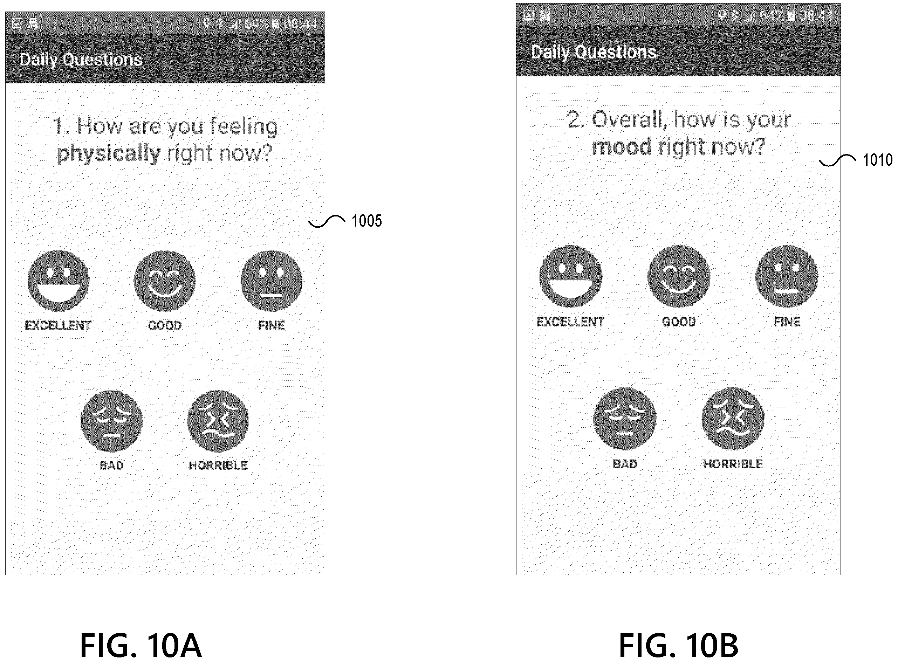

In some embodiments, the diagnostic tasks may include daily questions to assess the mood and physical health of the patient at the time of the active testing. The patient's response to the daily questions provide an assessment of the patient's daily mood fluctuations and may be used as a control when assessing symptoms associated with motor, cognitive and behavioral functions of the patient. FIGS. 10A and 10B depict example screenshots 1005 and 1010 illustrating examples of daily questions according to some embodiments. FIG. 10A depicts an example screenshot 1005 displaying an example daily question "How are you feeling physically right now?" The example screenshot 1005 also shows displayed icons labelled "Excellent," "Good," "Fine," "Bad," and "Horrible." The subject 110 chooses an answer by selecting the icon that best describes their health at that time. FIG. 10AB depicts an example screenshot 1010 displaying another example daily question "Overall, how is your mood right now?" The example screenshot 1010 also shows icons labelled "Excellent," "Good," "Fine," "Bad," and "Horrible." The subject 110 chooses an answer by selecting the icon that best describes their mood at that time.

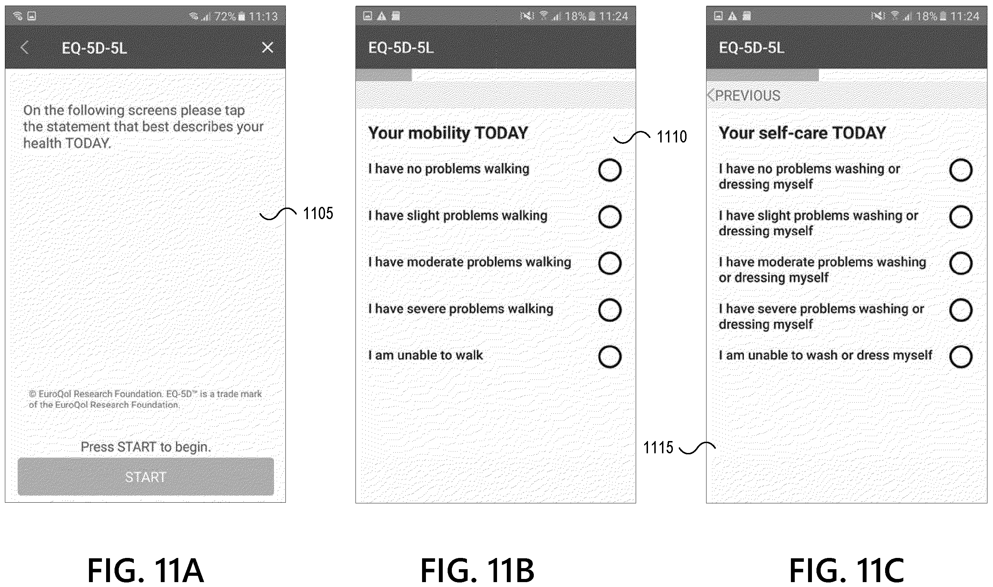

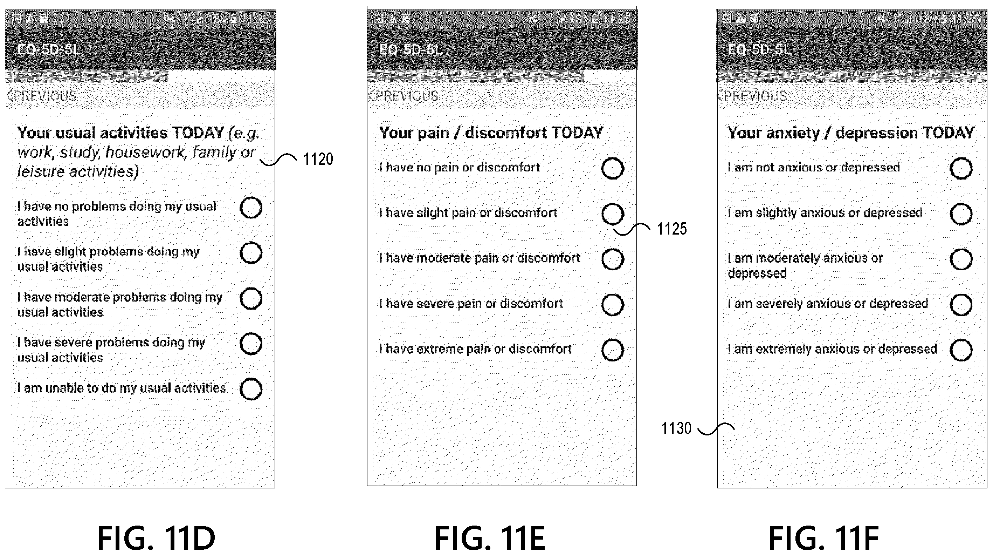

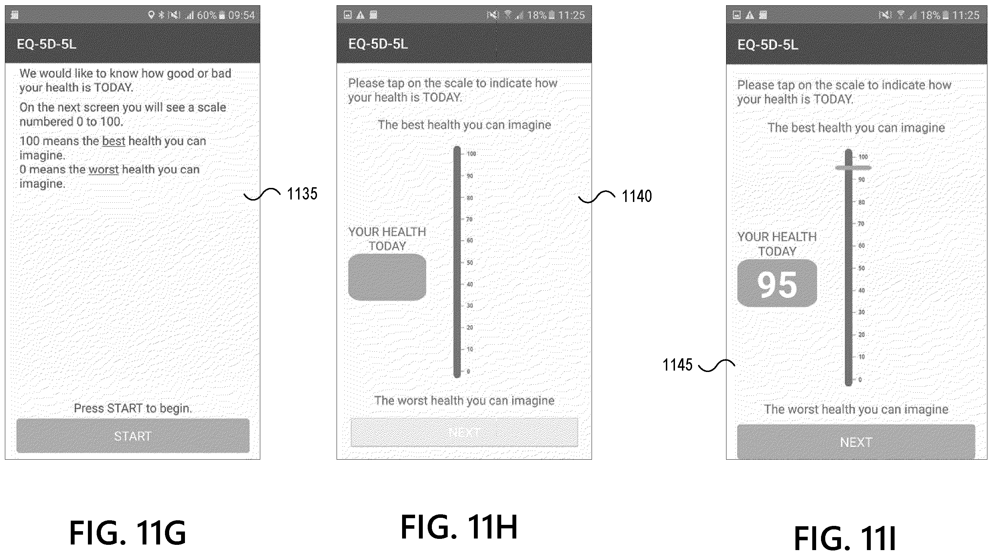

In some embodiments, the diagnostic tasks may implement a standardized test for measuring generic health status such as the EQ-5D-5L. FIGS. 11A-11I depict example screenshots 1105, 1110, 1115, 1120, 1125, 1130, 1135, 1140 and 1145 illustrating an example EQ-5D-5L test according to one or more illustrative aspects described herein. FIG. 11A is an example screenshot 1105 displaying general instructions for an example EQ-5D-5L test. FIG. 11B is an example screenshot 1110 displaying various statements related to mobility, such as, "I have no problems walking," "I have slight problems walking," etc. The subject 110 is instructed to select the statement that best describes the mobility of the subject 110. FIG. 11C is an example screenshot 1115 displaying statements related to self-care, such as, "I have no problems washing or dressing myself," "I have slight problems washing or dressing myself," etc. The subject 110 is instructed to select the statement that best describes the self-care of the subject 110. FIG. 11D is an example screenshot 1120 displaying statements related to usual activities (e.g. work, study, housework, family or leisure activities) such as "I have no problems doing my usual activities," "I have slight problems doing my usual activities," etc. FIG. 11E is an example screenshot 1125 related to the pain and discomfort experienced by the subject 110 and includes statements such as "I have no pain or discomfort," "I have slight pain or discomfort," etc. FIG. 11F is an example screenshot 1130 displaying various statements related to anxiety and depression of the subject 110. FIGS. 11G-11I depict example screenshots 1135, 1140 and 1145 illustrating some example questions related to the overall health of the subject 110.

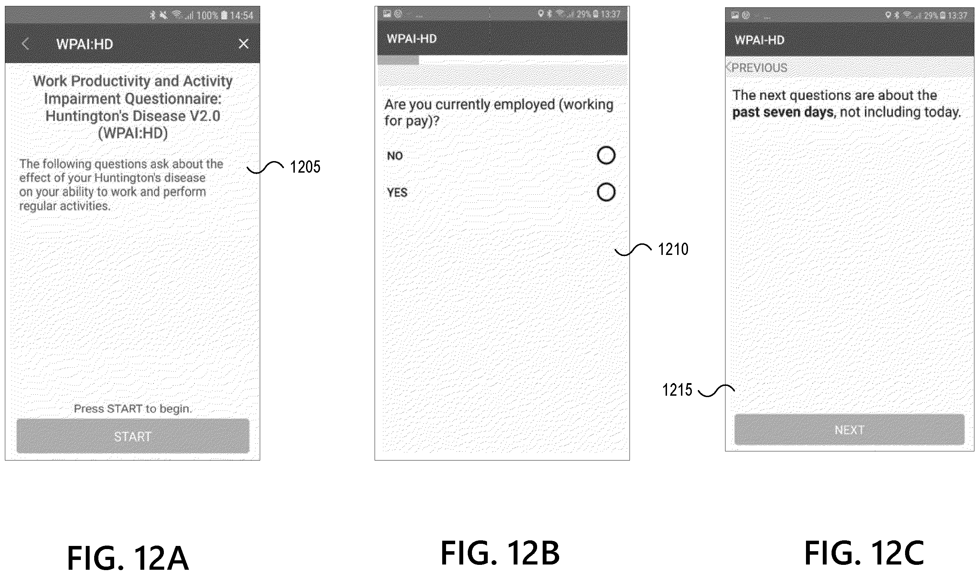

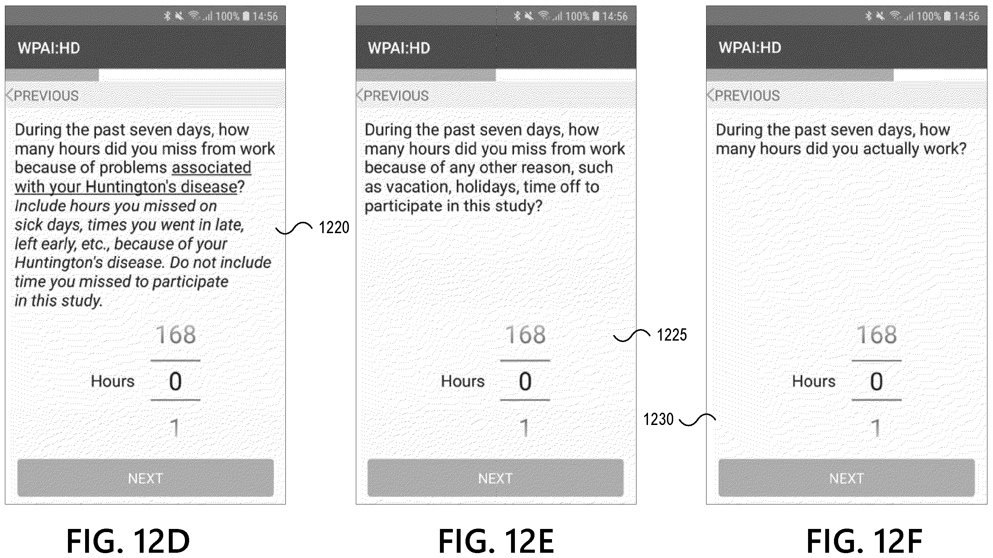

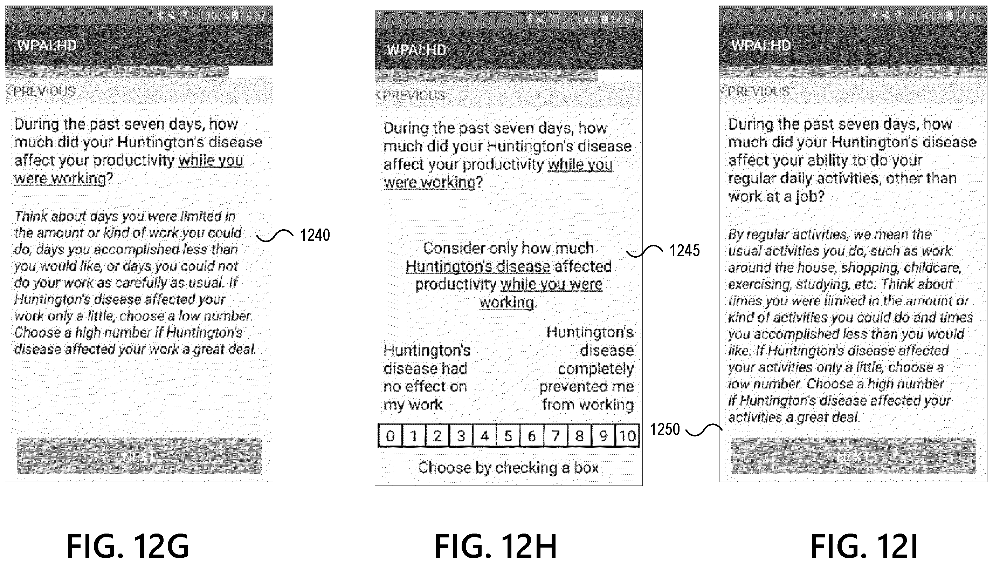

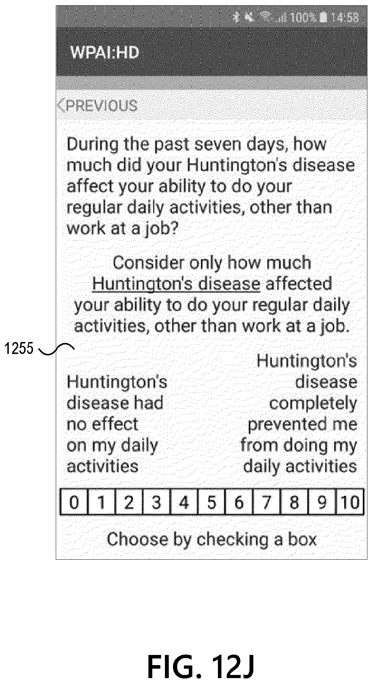

In some embodiments, the diagnostic tasks may implement a Work Productivity and Activity Impairment Questionnaire specific to Huntington's disease (WPAI-HD). The diagnostic tasks associated with WPAI-HD measure the effect of Huntington's disease on the subject's ability to work and perform regular activities. FIGS. 12A-12J depict example screenshots 1205, 1210, 1215, 1220, 1225, 1230, 1235, 1240, 1245 and 1250 illustrating an example WPAI-HD according to one or more illustrative aspects described herein. In some embodiments, the diagnostic tasks may prompt the subject to provide information about how many hours of work the subject missed because of problems associated with Huntington's disease including hours missed on sick days, the number of times the subject went in late to work, the number of times the subject left early from work. In some embodiments, the diagnostic tasks may prompt the subject 110 to provide information about how many hours the subject missed in the past seven days for reasons other than problems associated with Huntington's disease, such as, vacation, holidays, and time off to participate in a Huntington's disease study. In some embodiments, the diagnostic tasks may prompt the subject 110 to provide information about the number of hours the subject actually worked in the past seven days. In some embodiments, the diagnostic tasks may prompt the subject 110 to provide information about how much the symptoms of Huntington's disease affected productivity while the subject was working. In some embodiments, the diagnostic tasks may prompt the subject to provide information about how much the symptoms of Huntington's disease affected the subject's ability to perform regular daily activities unrelated to the subject's employment. Regular daily activities may include activities such as work around the house, shopping, childcare, exercising, studying.

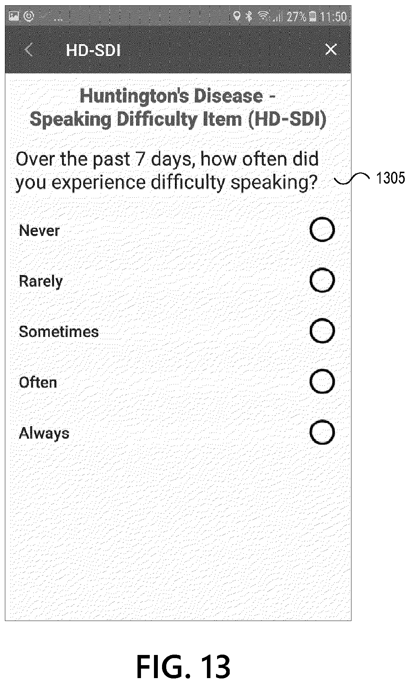

In some embodiments, the diagnostic tasks implement a Huntington's disease Speaking Difficult Item (HD-SDI). The diagnostic tasks associated with a HD-SDI test measure the effect of Huntington's disease on the subject's ability to speak. FIG. 13 depicts an example screenshot 1305 illustrating an example HD-SDI test according to some embodiments. In some embodiments, the subject is prompted to provide information about how often the subject experienced difficult speaking recently.

In some embodiments, the diagnostic tasks may implement a speeded tapping test. In some embodiments, the diagnostic tasks prompt the subject 110 to tap the display screen 160 of the device 105 as fast and regularly as possible, using the index finger of both the left and right hands. The speeded tapping test measures the speed of finger movements. In some embodiments, the diagnostic tasks associated with the speed tapping test may assess symptoms of bradykinesia, chorea and/or dystonia. In some embodiments, the diagnostic tasks associated with the speed tapping test are modelled on tapping tests that have been shown to be sensitive to symptom changes in early Huntington's disease (Bechtel et al. 2010; Tabrizi et al. 2012). A similar finger tapping task is also included as part of the Unified Huntington's Disease Rating. Scale (UHDRS) assessment (Huntington's Study Group 1996).

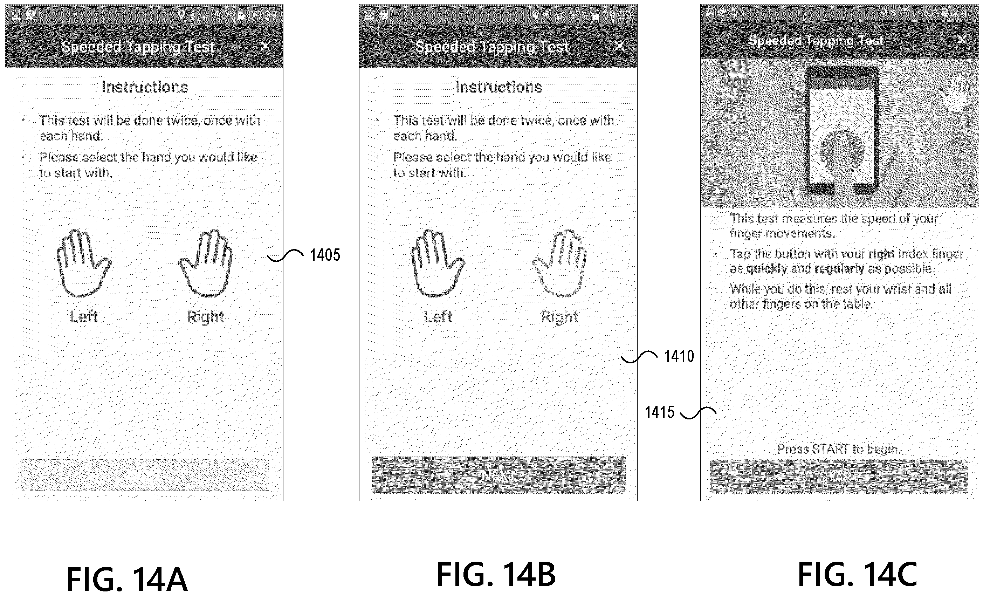

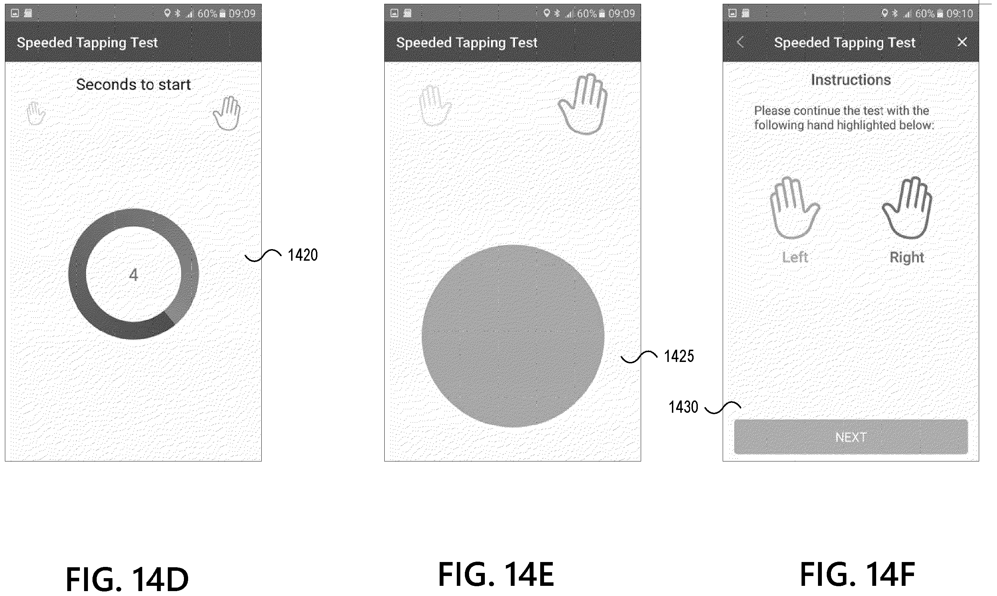

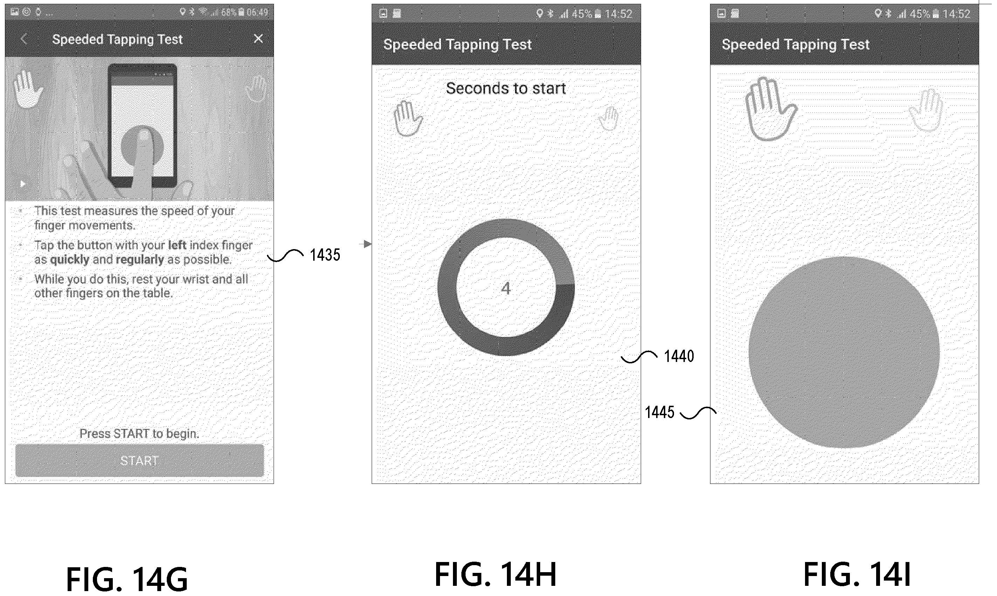

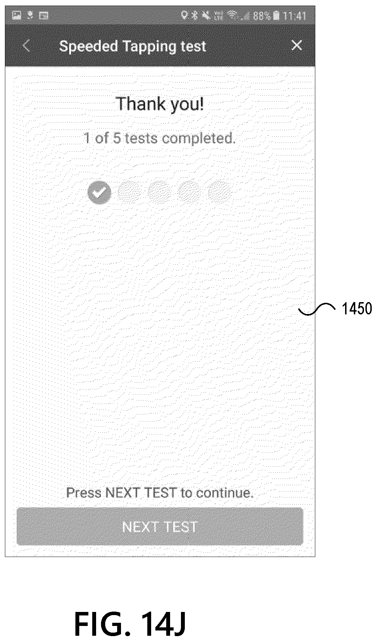

FIGS. 14A-14J depict example screenshots 1405, 1410, 1415, 1420, 1425, 1430, 1435, 1440, 1445 and 1450 illustrating an example speeded tapping test according to one or more illustrative aspects described herein. As shown in the screenshot 1405 of FIG. 14A, the subject is informed that the test is to be performed twice, once with each hand and the subject is prompted to select a hand to start with. As shown in the screenshot 1410 of FIG. 14B, the subject 110 selects the right hand. As shown in the screenshot 1415 of FIG. 14C, the subject is instructed to tap a button displayed on the screen 160 of the device 105 with the right index finger as quickly and regularly as possible while resting the wrist and other fingers on the table. Next, as shown in the screenshot 1420 of FIG. 14D, a countdown to the start of the test is displayed. The test begins and as shown in the screenshot 1425 of FIG. 14E, a button is displayed on the screen 160 of the device 105. The subject 110 taps the button on the screen 160 of the device 105 with the right index finger as quickly and regularly as possible. Next, as shown in the screenshot 1430 of FIG. 14F, the subject 110 is prompted to continue the test with the left hand. As shown in the screenshot 1435 of FIG. 14G, the subject is instructed to tap a button displayed on the screen 160 of the device 105 with the right index finger as quickly and regularly as possible while resting the wrist and other fingers on the table. As shown in the screenshot 1440 of FIG. 14H, a countdown to the start of the test is displayed. The test starts and as shown in the screenshot 1445 of FIG. 14I, a button is displayed on the screen 160 of the device 105. The subject 110 taps the button on the screen 160 with the right index finger as quickly and regularly as possible. Then, as shown in the screenshot 1450 of FIG. 14J, the test ends and the subject 110 is informed that the speeded tapping test is complete.

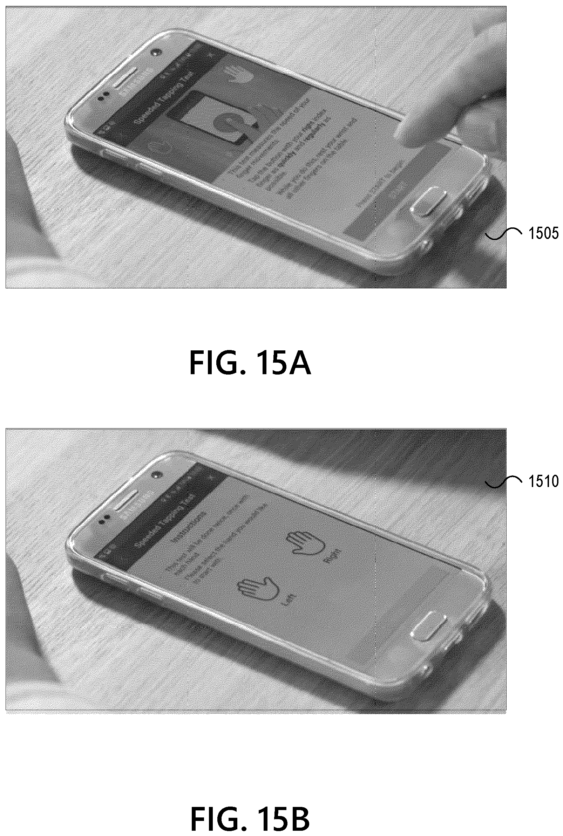

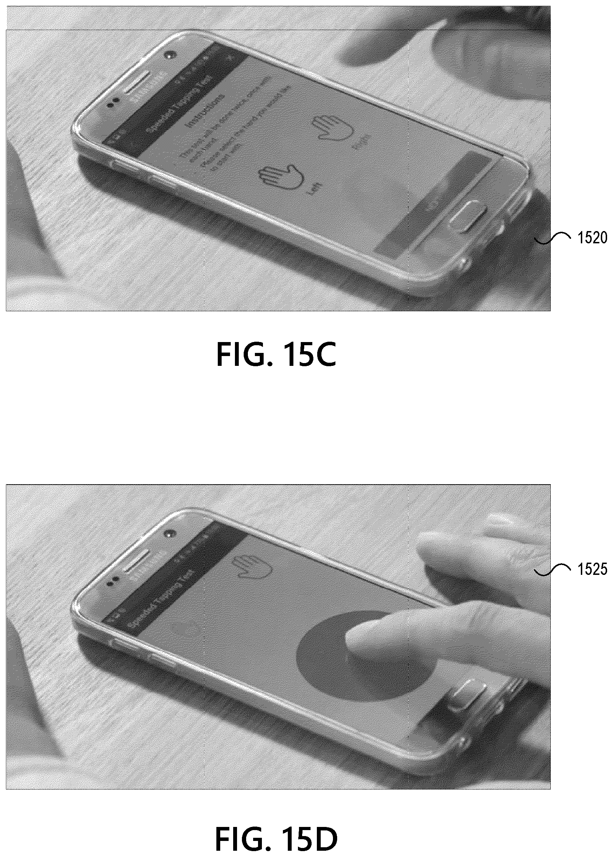

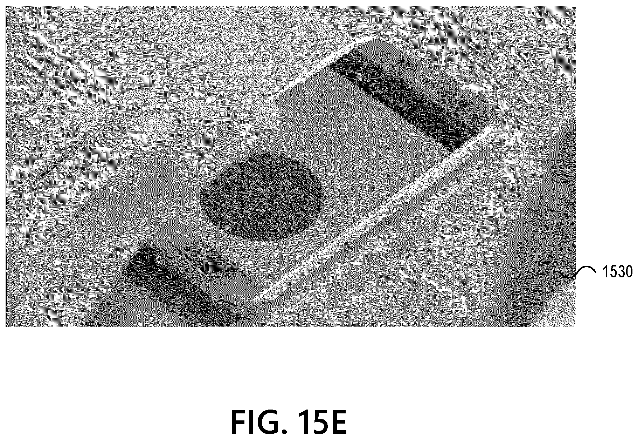

In some embodiments, the diagnostic application may include an instructional video for the speeded tapping test that the subject may view on the display screen 160 of the device 105. FIGS. 15A-15E depict example screenshots 1505, 1510, 1515, 1520, and 1525 from an instructional video for an example speeded tapping test according to one or more illustrative aspects described herein.

In some embodiments, the diagnostic tasks may implement a draw a shape test that prompt the subject 110 to trace a series of increasingly complex shapes on the display screen 160 of the device 105. In some embodiments, the shapes may include lines, a square, a circle, an eight, and a spiral. This test is designed to assess visuo-motor coordination and fine motor impairment in early Huntington's disease patients. The diagnostic tasks are modelled on circle tracing tasks that have been shown to be sensitive to symptom changes in the early stages of Huntington's disease (Say et al. 2011; Tabrizi et al. 2013).

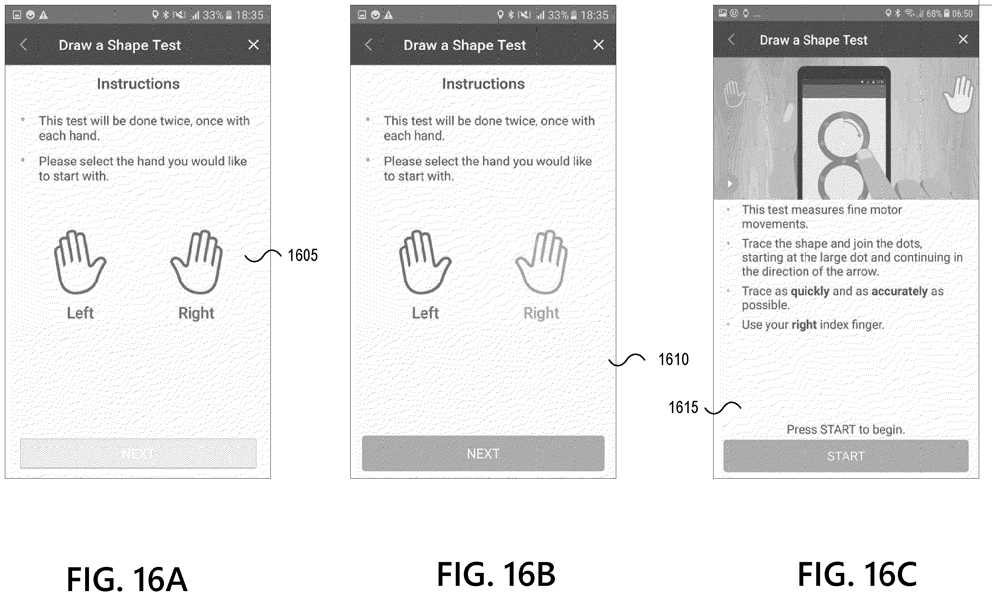

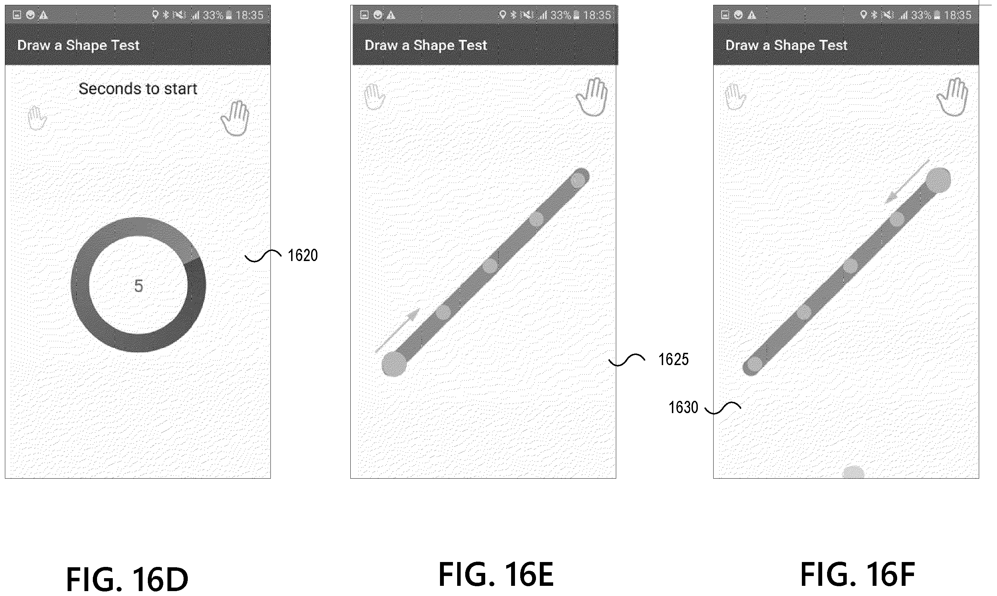

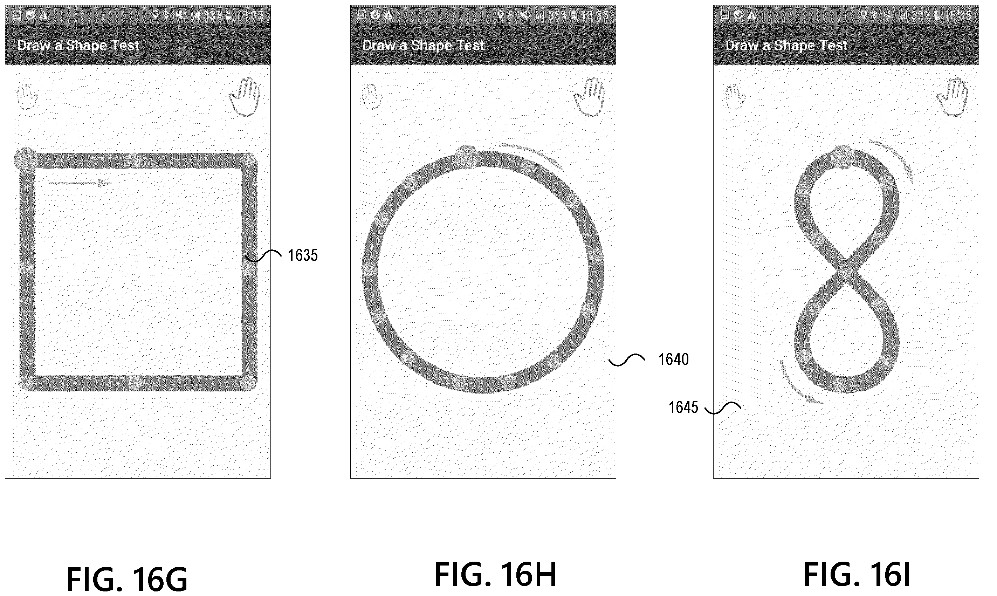

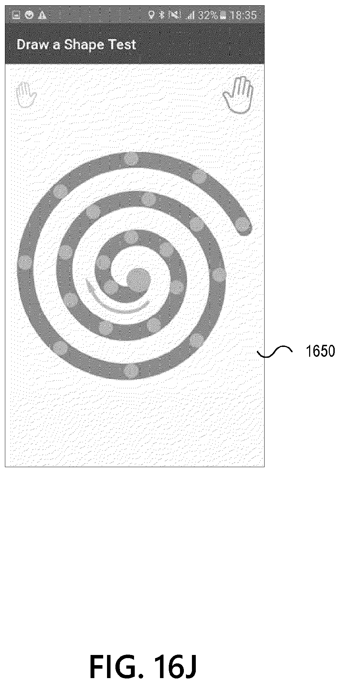

FIGS. 16A-16T depict example screenshots 1605, 1610, 1615, 1620, 1625, 1630, 1635, 1640, 1645, 1650, 1655, 1660, 1665, 1670, 1675, 1680, 1685, 1690, 1695 and 1698 illustrating an example draw a shape test according to one or more illustrative aspects described herein. As shown in the screenshot 1605 of FIG. 16A, the subject 110 is informed that the test will be done twice, once with each hand. The subject 110 is prompted to select the hand to start with. As shown in the screenshot 1610 of FIG. 16B, the subject 110 selects the right hand to start with. As shown in the screenshot 1615 of FIG. 16C, the subject 110 is informed that the test measures fine motor movements. The subject 110 is instructed to trace a displayed shape and join the dots displayed along the outline of the shape, continuing in the direction of an arrow. The subject 110 is instructed to trace as quickly and as accurately as possible using the right index finger. Next, as shown in the screenshot 1620 of FIG. 16D, a countdown to the start of the test is shown. Once the test begins, a shape is displayed on the screen. As shown in the screenshot 1625 of FIG. 16E, a line is displayed. Several dots are also displayed along the line. Also displayed is an arrow indicating the direction in which the subject 110 should trace the shape along the dots. Using the right index finger, the subject 110 traces the line in the direction of the arrow. Next, as shown in the screenshot 1630 of FIG. 16F, a line, dots and arrow are displayed as in the screenshot 1625 of FIG. 16E. However, the arrow is displayed in an opposite direction from the arrow displayed in the screenshot 1625 of FIG. 16E. Using the right index finger, the subject 110 traces the line in the direction of the arrow. Next, as shown in the screenshot 1635 of FIG. 16G, an outline of a square with dots and an arrow are displayed. Using the right index finger, the subject 110 traces the square shape in the direction of the arrow. Next, as shown in the screenshot 1640 of FIG. 16H, an outline of a circle with dots and an arrow are displayed. Using the right index finger, the subject 110 traces the circle in the direction of the arrow. Next, as shown in the screenshot 1645 of FIG. 16I, an outline of a shape similar to the number eight with dots and an arrow are displayed. Using the right index finger, the subject 110 traces the shape in the direction of the arrow. Next, as shown in the screenshot 1650 of FIG. 16J, an outline of a spiral shape with dots and an arrow are displayed. Using the right index finger, the subject 110 traces the shape in the direction of the arrow.

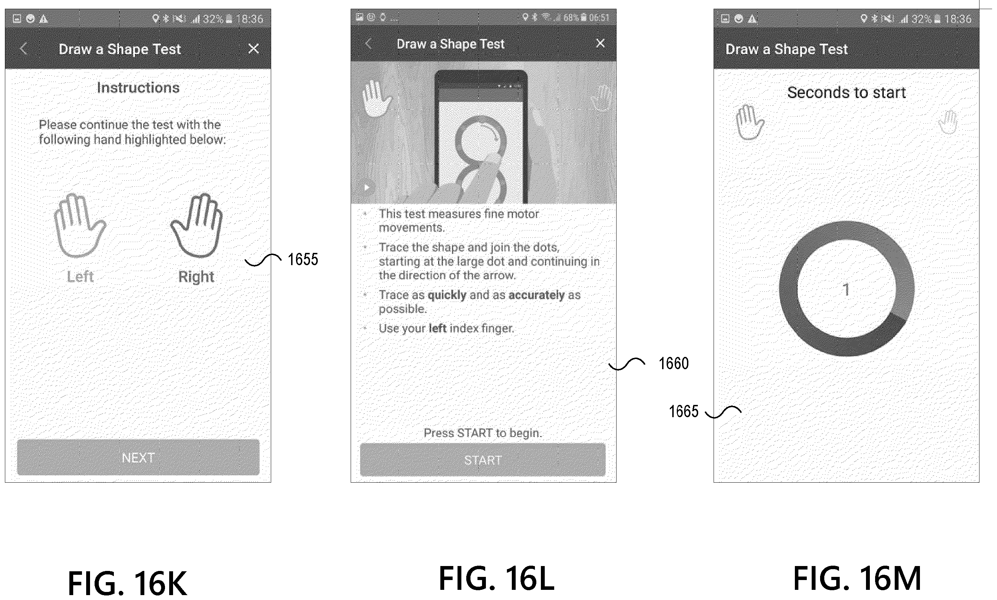

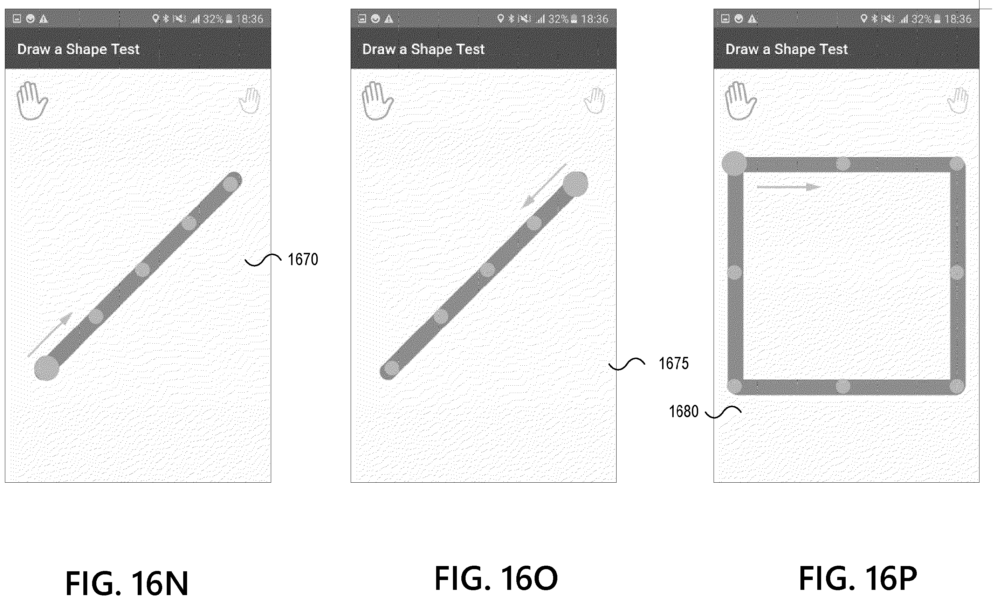

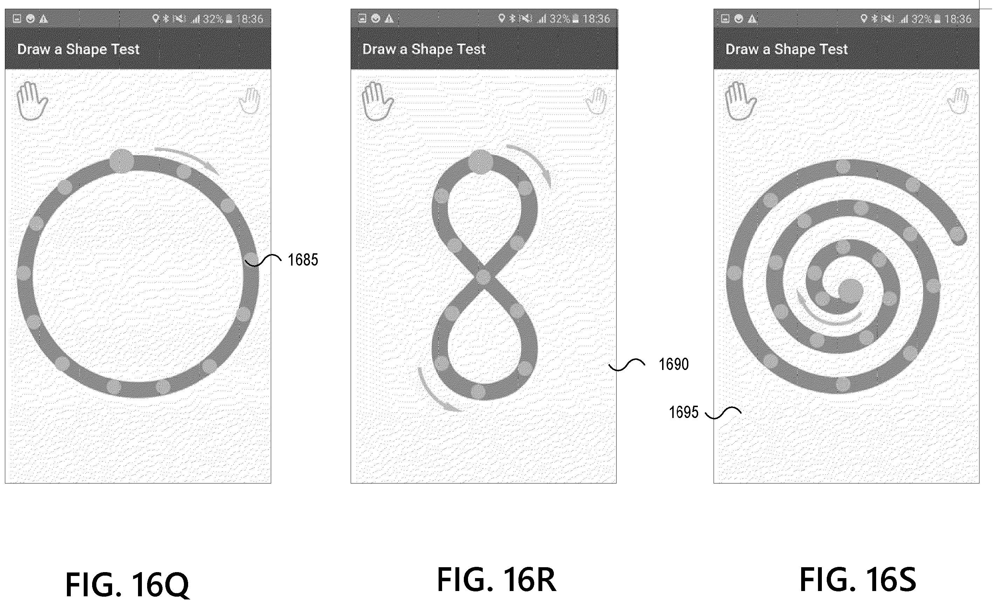

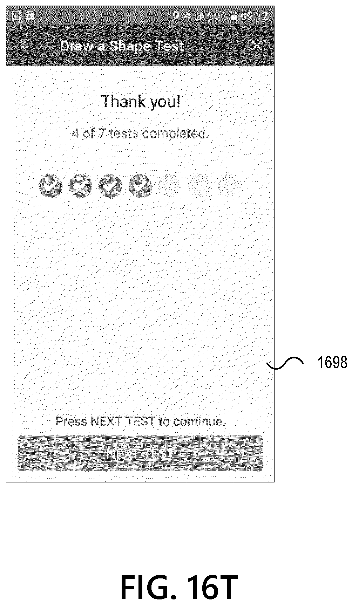

The test is repeated with the left hand. As shown in the screenshot 1655 of FIG. 16K, the subject 110 is instructed to continue the test with the left hand. As shown in the screenshot 1660 of FIG. 16L, the subject 110 is informed that the test measures fine motor movements. The subject 110 is instructed to trace a displayed shape and join the dots displayed along the outline of the shape, continuing in the direction of an arrow. The subject 110 is instructed to trace as quickly and as accurately as possible using the left index finger. Next, as shown in the screenshot 1665 of FIG. 16M, a countdown for beginning the test is shown. Once the test begins, a shape is displayed on the screen. As shown in the screenshot 1670 of FIG. 16N, a line is displayed. Several dots are also displayed along the outline of the line. Also displayed is an arrow indicating the direction in which the subject 110 should trace the shape. Using the right index finger, the subject 110 traces the line in the direction of the arrow. Next, as shown in the screenshot 1675 of FIG. 16O, a line, dots and arrow are displayed as in the screenshot 1670 of FIG. 16N. However, the arrow is displayed in an opposite direction from the arrow displayed in the screenshot 1670 of FIG. 16N. Using the left index finger, the subject 110 traces the line in the direction of the arrow. Next, as shown in the screenshot 1680 of FIG. 16P, an outline of a square with dots and an arrow are displayed. Using the left index finger, the subject 110 traces the square shape in the direction of the arrow. Next, as shown in the screenshot 1685 of FIG. 16Q, an outline of a circle with dots and an arrow are displayed. Using the left index finger, the subject 110 traces the circle in the direction of the arrow. Next, as shown in the screenshot 1690 of FIG. 16R, an outline of a shape similar to the number eight with dots and an arrow are displayed. Using the right index finger, the subject 110 traces the shape in the direction of the arrow. Next, as shown in the screenshot 1695 of FIG. 16S, an outline of a spiral shape with dots and an arrow are displayed. Using the right index finger, the subject 110 traces the shape in the direction of the arrow. Next, as shown in the screenshot 1698 of FIG. 16T, the subject 110 is informed that the test is complete.

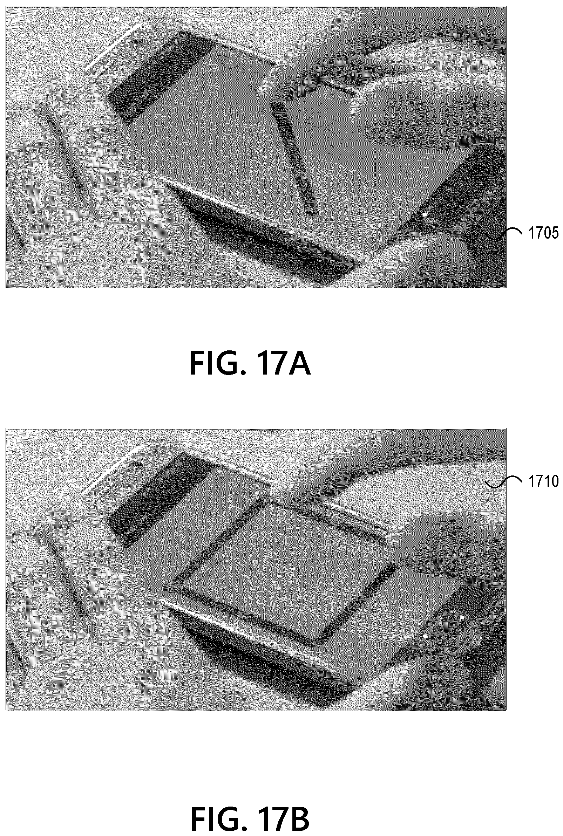

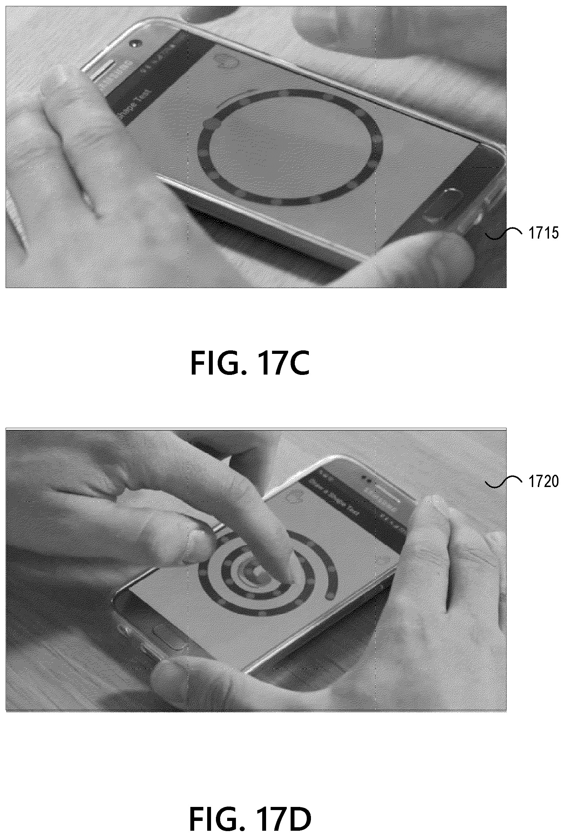

In some embodiments, the diagnostic application may include an instructional video for the draw a shape test that the subject can view on the display screen 160 of the device 105. FIGS. 17A-17E depict example screenshots 1705, 1710, 1715, 1720 and 1725 from an example instructional video for a draw a shape test according to one or more illustrative aspects described herein.

In some embodiments, the diagnostic tasks include a chorea test in which the subject 110 is prompted to hold the device 105 still in one hand with the corresponding arm outstretched, while wearing a wrist-worn wearable, such as the second sensor 120b shown in FIG. 1. As a dual task, the subject 110 is also prompted to count backwards aloud. To ensure correct execution, the voice of the subject 110 is recorded. This test is designed to assess chorea and draws on other sensor-based approaches to measure chorea (Reilmann et al. 2010, 2011; Kegelmeyer et al. 2017). A chorea assessment is also part of the UHDRS (Huntington's Study Group 1996).

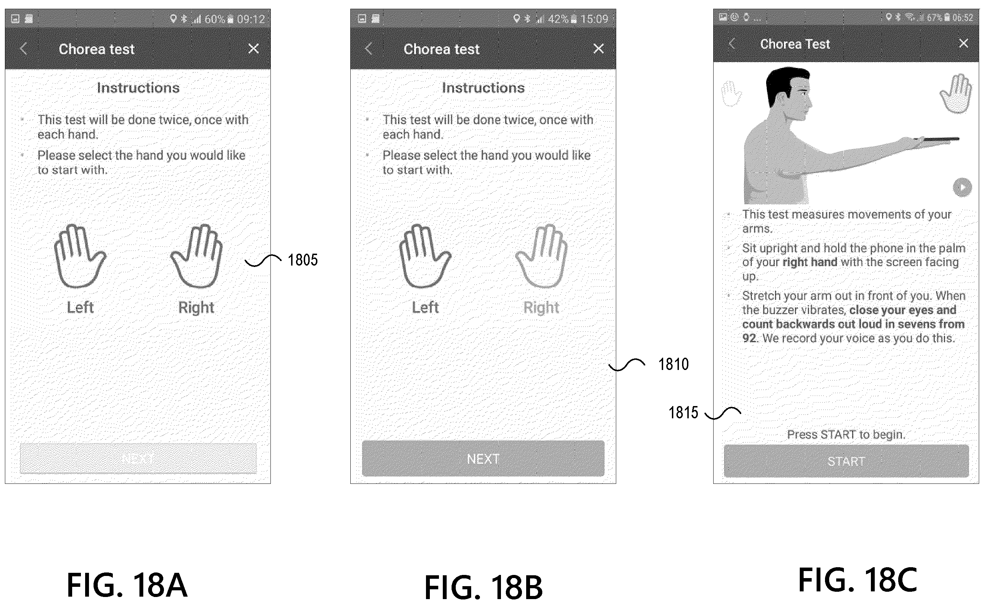

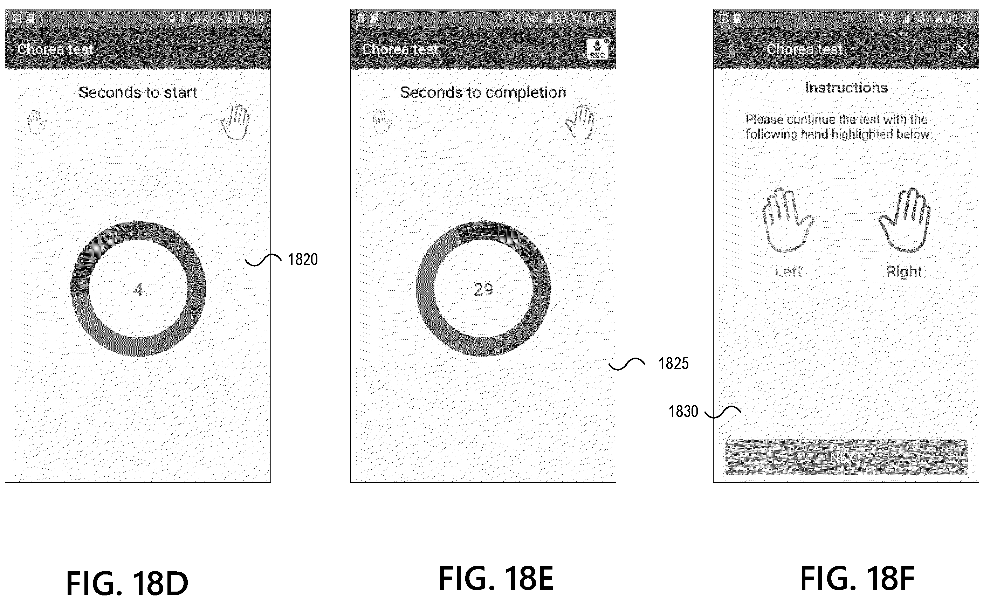

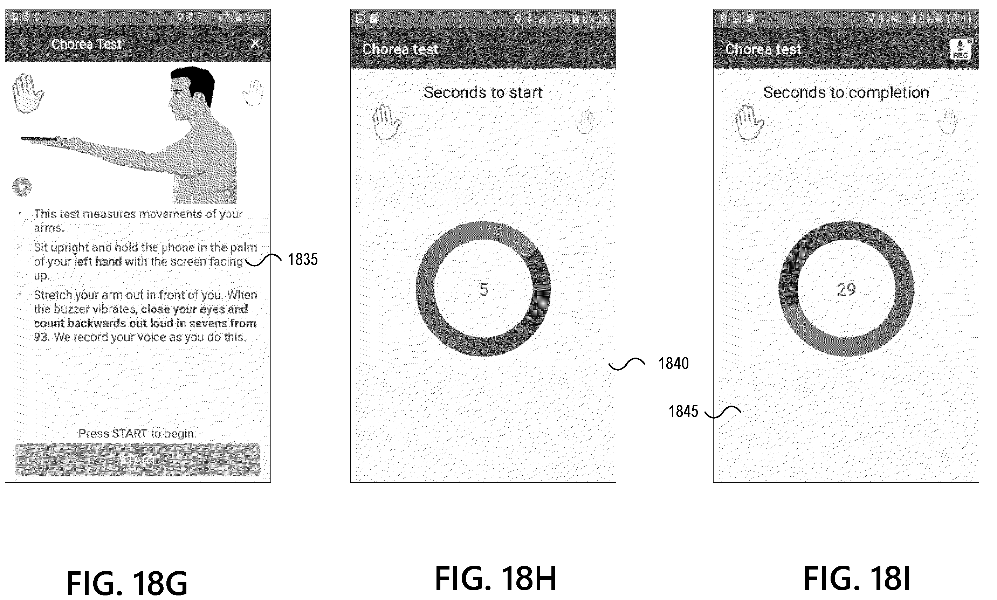

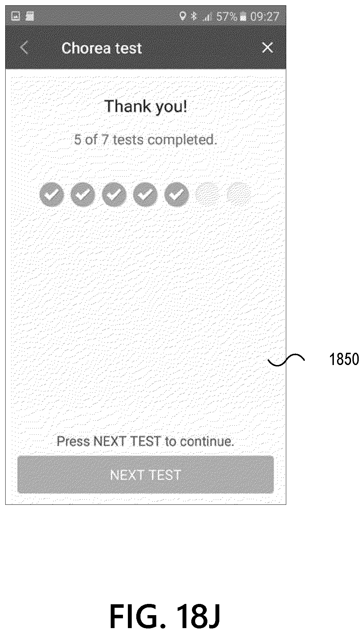

FIGS. 18A-18J depict example screenshots 1805, 1810, 1815, 1820, 1825, 1830, 1835, 1840, 1845, and 1850 illustrating an example chorea test according to one or more illustrative aspects described herein. As shown in the screenshot 1805 of FIG. 18A, the subject 110 is informed that the test will be performed twice, once with each hand. The subject 110 is prompted to select the hand to start with. As shown in the screenshot 1810 of FIG. 18B, the subject 110 selects the right hand to start with. As shown in the screenshot 1815 of FIG. 16C, the subject 110 is informed that the test measures movements of the arms. The subject 110 is instructed to sit upright and hold the device 105 in the palm of the right hand with the display screen 160 of the device 105 facing up. The subject 110 is instructed to stretch the right arm out in front. The subject 110 is also instructed that when the buzzer of the phone vibrates, to close the eyes and count backwards out loud in sevens from 92. The subject's voice is recorded. Next, as shown in the screenshot 1820 of FIG. 18D, a countdown to the start of the test is shown. Once the test begins, a countdown to completion of the test is displayed, as shown in the screenshot 1825 of FIG. 18E. The test is repeated using the left hand of the subject 110. FIGS. 18F-18I depict example screenshots 1825, 1830, 1835, 1840, and 1845 illustrating the test for the left hand. Then, as shown in the screenshot 1850 of FIG. 18J, the subject 110 is informed that the test is complete.

In some embodiments, the subject 110 the diagnostic application may include an instructional video for the chorea test that the subject can view on the display screen 160 of the device 105. 19A-19E depict example screenshots 1905, 1910, 1915, 1920 and 1925 from an example instructional video for a chorea test according to one or more illustrative aspects described herein.

In some embodiments, the diagnostic tasks implement a balance test. The subject 110 is instructed to stand still while wearing the device 105 and the wrist-worn wearable, such as sensor 120b shown in FIG. 1. The balance test assesses the static balance function of the subject 110. Sensor-based approaches for measuring static balance have been shown to be sensitive to differences in symptoms in early Huntington's disease (Dalton et al. 2013). The test is also part of established scales like the Berg Balance Scale (Berg et al. 1992), which are used in HD (Busse et al. 2009; Rao et al. 2009). The test is anchored to the UHDRS assessments for maximal dystonia, maximal chorea, and tandem walking (Huntington Study Group 1996).

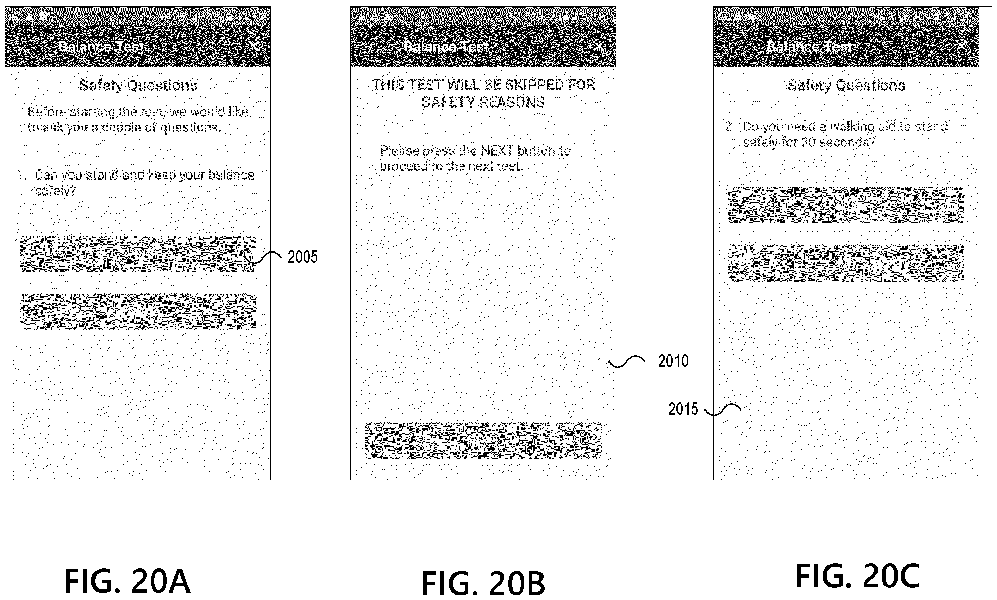

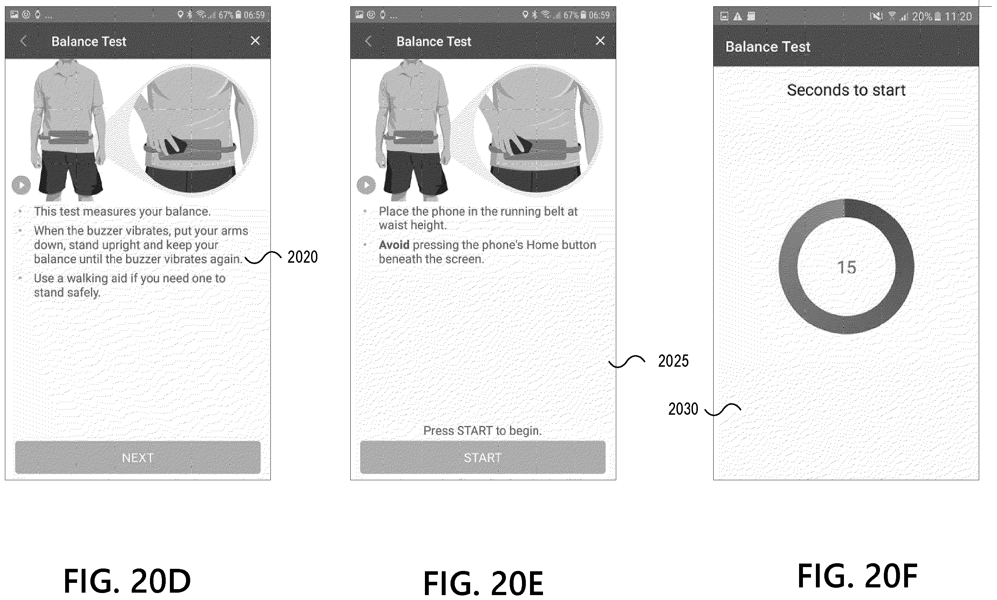

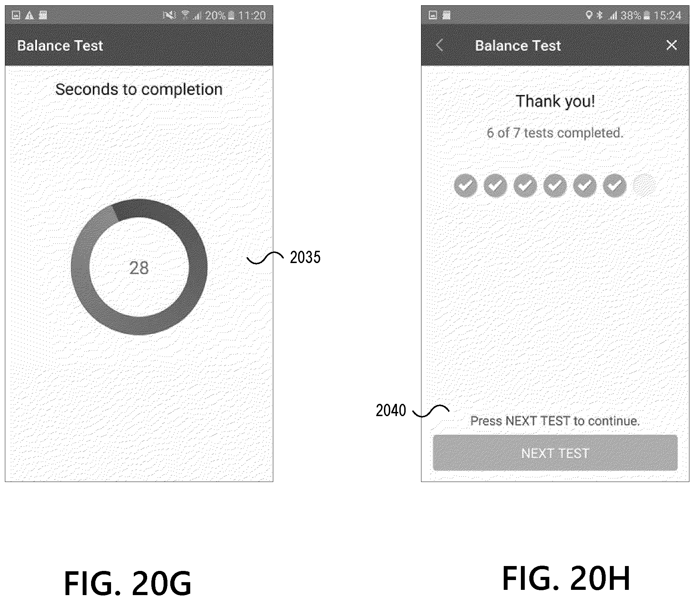

FIGS. 20A-20H depict example screenshots 2005, 2010, 2015, 2020, 2025, 2030, 2035, and 2040 illustrating an example balance test according to one or more illustrative aspects described herein. Before prompting the subject to perform the balance test, the subject 110 is instructed to answer several questions to assess whether it is safe for the subject 110 to perform the balance test. As shown in the screenshot 2005 of FIG. 20A, the subject 110 is asked whether the subject can stand and keep their balance safely. If the subject 110 selects the answer "No," the subject 110 is informed that the test will be skipped for safety reasons, as shown in the screenshot 2010 of FIG. 20B. If the subject 110 selects the answer "Yes," then, as shown in the screenshot 2015 of FIG. 20C, the subject 110 is asked whether the subject needs a walking aid to stand safely for 30 seconds. The subject selects "Yes" or "No." Next, as shown in the screenshot 2020 of FIG. 20D, the subject is informed that the test measures balance. The subject is instructed to when the buzzer vibrates, to put both arms down, stand upright and maintain balance until the buzzer vibrates again. The subject is instructed to use a walking aid if needed to stand safely. Next, as shown in the screenshot 2025 of FIG. 20E, the subject is instructed to place the phone in a running belt at waist height. The subject is also instructed to avoid pressing the phone's "Home" button. Next, as shown in the screenshot 2030 of FIG. 20F, a countdown to the start of the test is displayed. The buzzer of the phone vibrates and the test is started. As shown in the screenshot 2035 of FIG. 20G, a countdown to the end of the test is displayed. The subject performs the balance test as instructed by putting both arms down, standing upright and maintaining balance until the buzzer vibrates again, ending the test. Then, as shown in the screenshot 2040 of FIG. 20H, the subject 110 is informed that the test is complete.

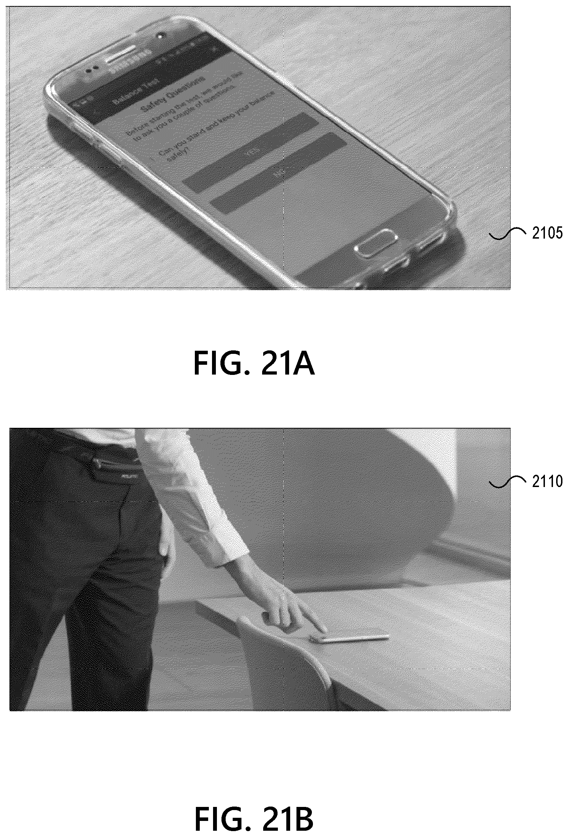

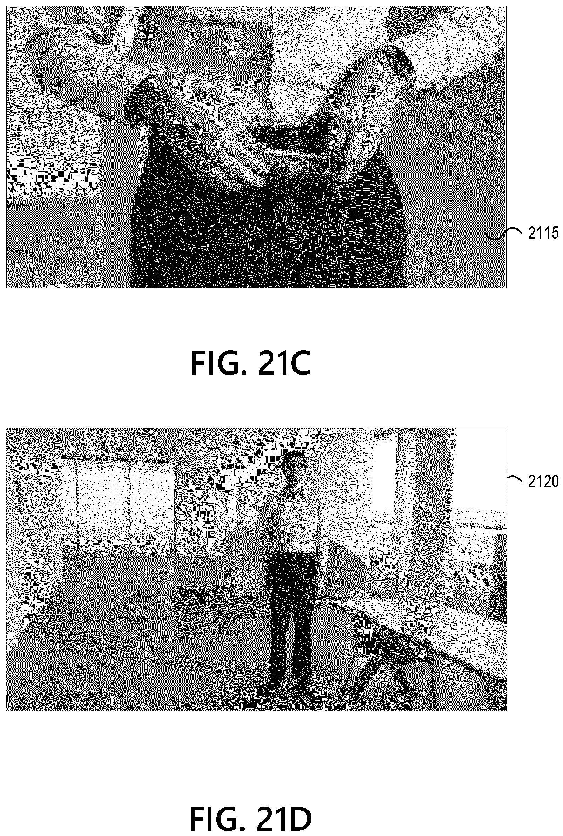

In some embodiments, the diagnostic application may include an instructional video for the balance test that the subject 110 may view on the display screen 160 of the device 105. FIGS. 21A-21D depict example screenshots 2105, 2110, 2115 and 2120 from an instructional video for an example balance test according to one or more illustrative aspects described herein.

In some embodiments, the diagnostic tasks may implement a u-turn test. The subject 110 is instructed to walk and turn safely between two points that are at least four steps apart, while wearing the device 105 and the wrist-worn wearable, such as the second sensor 120b shown in FIG. 1. In some embodiments, the subject 110 is instructed to make at least five turns. The test is designed to assess gait and lower-body bradykinesia, which are also assessed by the UHDRS. It is modelled on the Timed Up and Go Test, which has been clinically validated for the HD population (Busse et al. 2009; Rao et al. 2009). The test is anchored to the UHDRS gait, bradykinesia body, and tandem walking items (Huntington Study Group 1996).

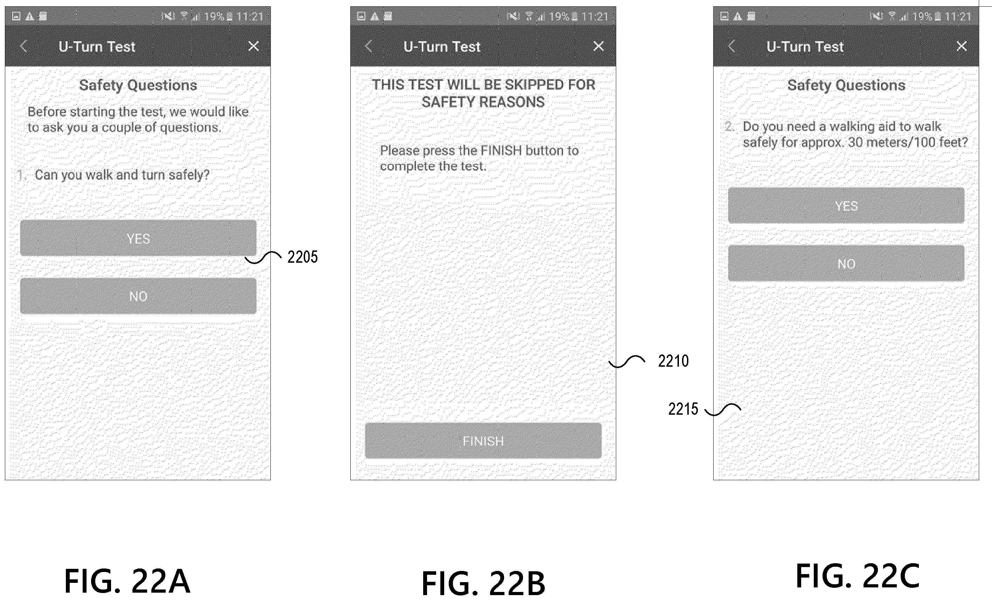

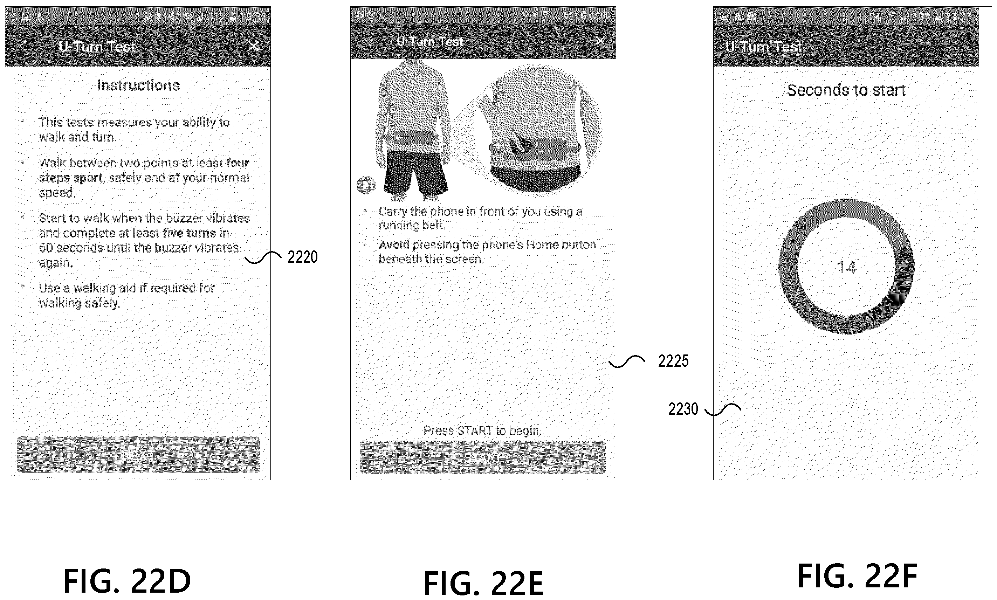

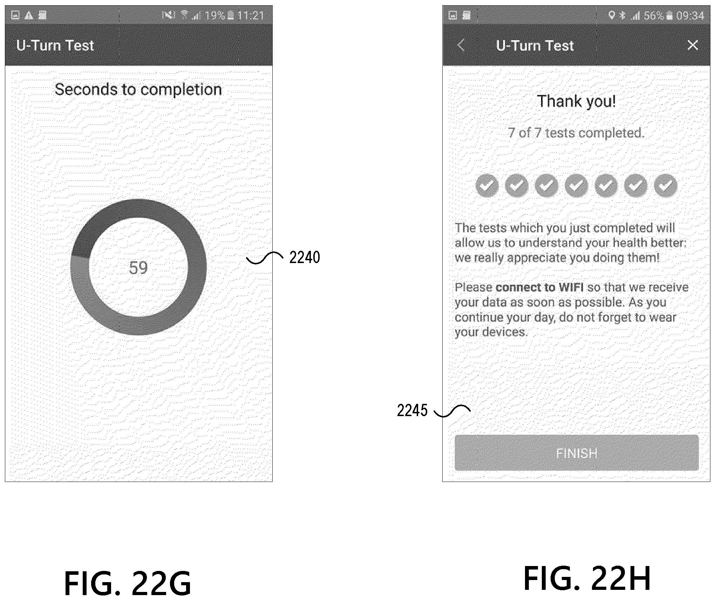

FIGS. 22A-22H depict example screenshots 2205, 2210, 2215, 2220, 2225, 2230, 2235, 2240, and 2245 illustrating an example u-turn test according to one or more illustrative aspects described herein. Before prompting the subject to perform the u-turn test, the subject 110 is instructed to answer several questions to assess whether it is safe for the subject 110 to actions associated with the u-turn test. As shown in the screenshot 2005 of FIG. 22A, the subject 110 is asked whether the subject can walk and turn safely. If the subject 110 selects the answer "No," the subject 110 is informed that the test will be skipped for safety reasons, as shown in the screenshot 2210 of FIG. 22B. If the subject 110 selects the answer "Yes," then, as shown in the screenshot 2215 of FIG. 20C, the subject 110 is asked whether the subject needs a walking aid to walk safely for approximately 30 meters or 100 feet. The subject is prompted to answer the question by selecting "Yes" or "No." Next, as shown in the screenshot 2220 of FIG. 22D, the subject is informed that the test the subject's ability to walk and turn. The subject is instructed to walk between two points at least four steps apart, safely at a speed that is normal for the subject. The subject is instructed to start walking when the buzzer vibrates and complete at least five turns in 60 seconds until the buzzer vibrates again. The subject is also instructed to use a walking aid if needed to walk safely. Next, as shown in the screenshot 2225 of FIG. 22E, the subject is instructed to carry the phone in front in a running belt at waist height. The subject is also instructed to avoid pressing the phone's "Home" button. Next, as shown in the screenshot 2230 of FIG. 22F, a countdown to the start of the test is displayed. The buzzer of the phone vibrates and the test begins. As shown in the screenshot 2240 of FIG. 22G, a countdown to the end of the test is displayed. The subject performs the actions of the u-turn test until the buzzer vibrates which ends the test. Then, as shown in the screenshot 2240 of FIG. 22H, the subject 110 is informed that the test is complete.

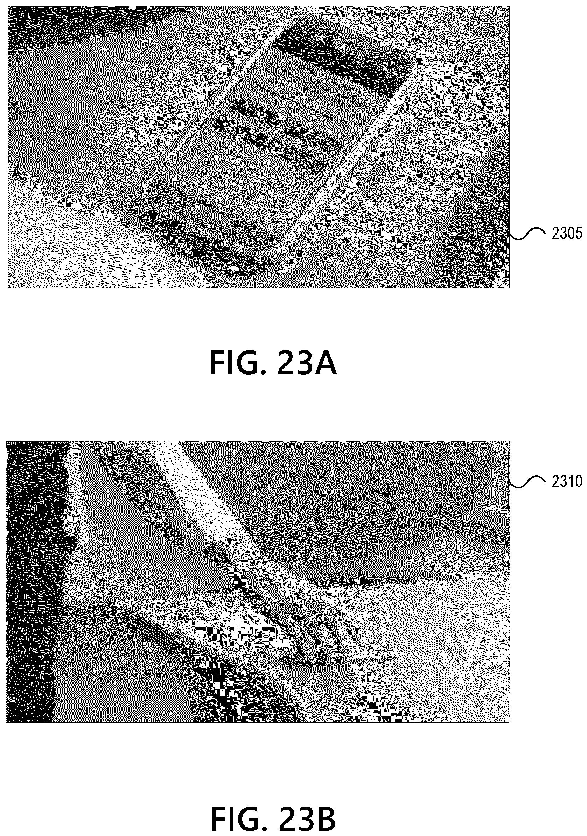

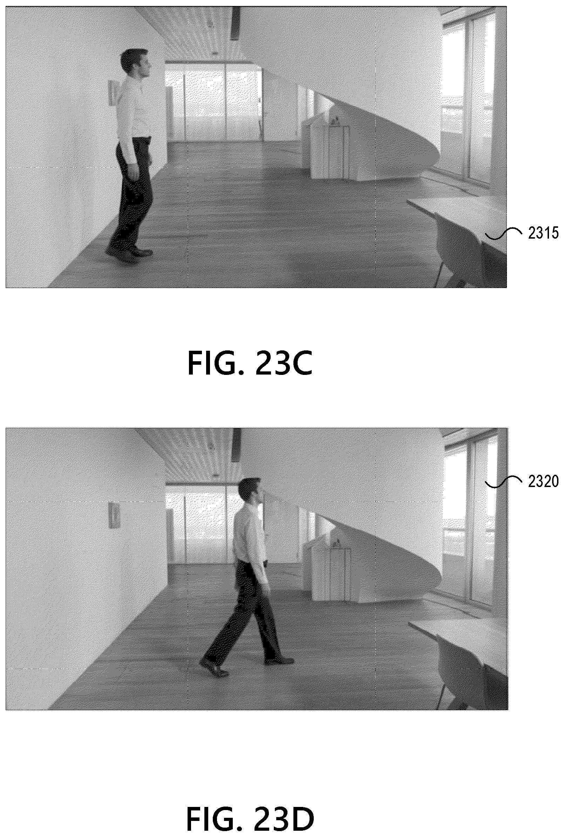

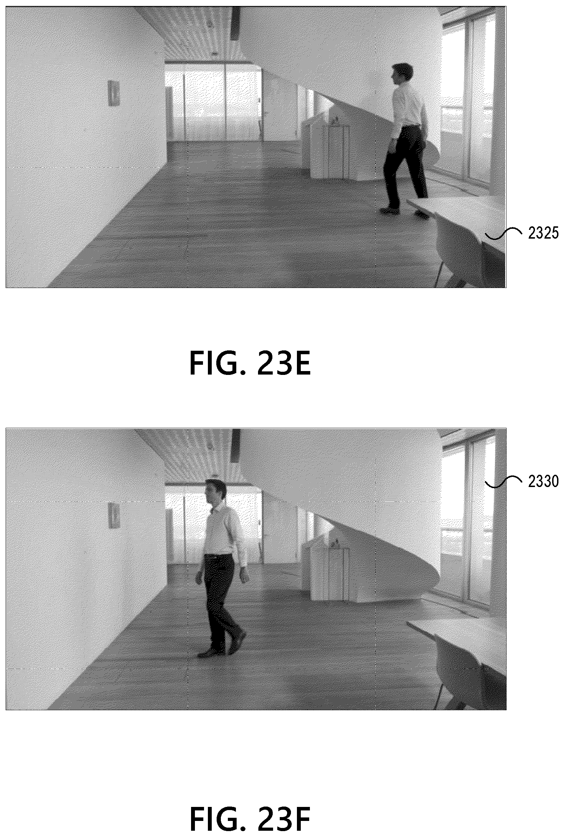

In some embodiments, the diagnostic application may include an instructional video for the u-turn test that the subject 110 may view on the display screen 160 of the device 105. FIGS. 23A-23F depict example screenshots 2305, 2310, 2315, 2320, 2325, and 2330 from an instructional video for an example u-turn test according to some embodiments.

In some embodiments, the diagnostic tasks may implement a walk test. The subject 110 is instructed to walk as fast as is safely possible for 200 meters or 2 minutes every day. Preferably, the test is performed in a straight path with no obstacles (e.g., in a park). Sensor-based approaches for measuring gait have been shown to be sensitive to differences in symptoms in early HD (Dalton et al. 2013). The test is anchored to the UHDRS gait, bradykinesia body, and tandem walking items (Huntington Study Group 1996).

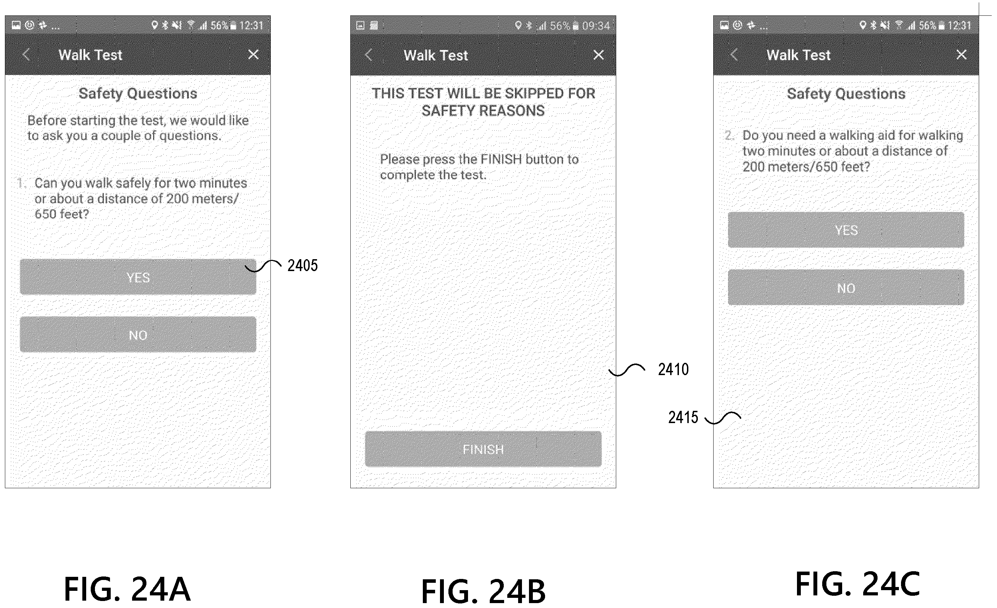

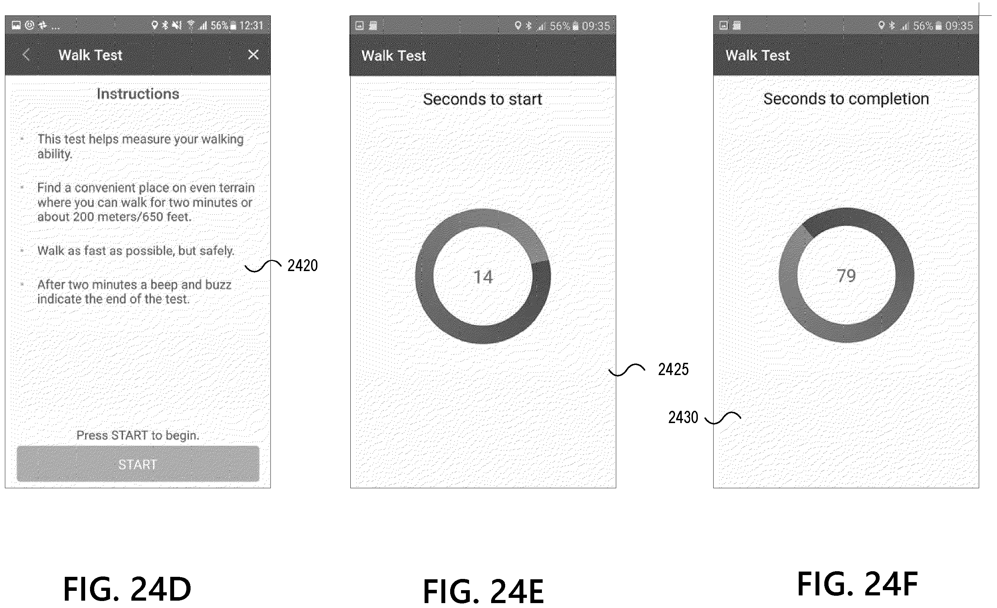

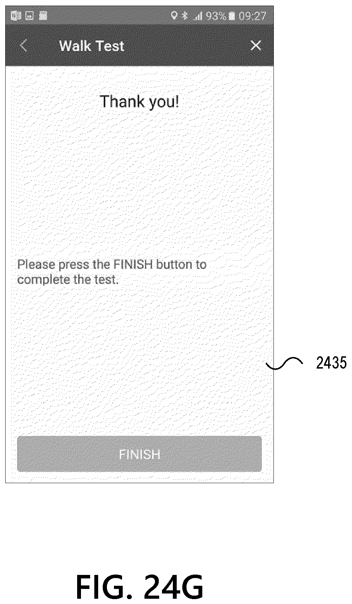

FIGS. 24A-24G depict example screenshots 2405, 2410, 2415, 2420, 2425, 2430, 2435, 2440, and 2445 illustrating an example walk test according to one or more illustrative aspects described herein. Before prompting the subject to perform the walk test, the subject 110 is instructed to answer several questions to assess whether it is safe for the subject 110 to perform the actions associated with the walk test. As shown in the screenshot 2405 of FIG. 24A, the subject 110 is asked whether the subject can walk safely of two minutes or about a distance of 200 meters or 650 feet. If the subject 110 answers "No," the subject 110 is informed that the test will be skipped for safety reasons, as shown in the screenshot 2410 of FIG. 24B. If the subject 110 selects the answer "Yes," then, as shown in the screenshot 2415 of FIG. 24C, the subject 110 is asked whether the subject needs a walking aid to walk safely for two minutes or about a distance of 200 meters or 650 feet. The subject is prompted to answer the question by selecting "Yes" or "No." Next, as shown in the screenshot 2420 of FIG. 24D, the subject is informed that the test helps measure the subject's ability to walk. The subject is instructed to find a convenient place on even terrain where the subject can walk for two minutes or about 200 meters (650 feet). The subject is instructed to safely walk as fast as possible. The subject is also informed that after two minutes, a beep and buzz by the device will indicate the end of the test. Next, as shown in the screenshot 2425 of FIG. 24E, a countdown to the start of the test is displayed. The test begins and as shown in the screenshot 2230 of FIG. 24F, a countdown to the end of the test is displayed. The subject performs the actions of the walk test. Then, as shown in the screenshot 2435 of FIG. 24G, the subject 110 is informed that the test is complete.

In some embodiments, the diagnostic tasks include a Symbol Digit Modalities Test (SDMT) that may be modelled on the pen and paper SDMT (Smith, 1968). The subject 110 is prompted to match symbols with numbers according to a key as quickly and accurately as possible. The key, symbols, numbers are displayed on the display screen 160 of the device 105. The SDMT test assesses visuo-motor integration, and measures visual attention and motor speed. The SDMT has been shown to be sensitive to symptom changes in early Huntington's disease patients (Tabrizi 2012) and is part of the Unified Huntington's Disease Rating Scale (UHDRS) assessment (Huntington's Study Group, 1996).

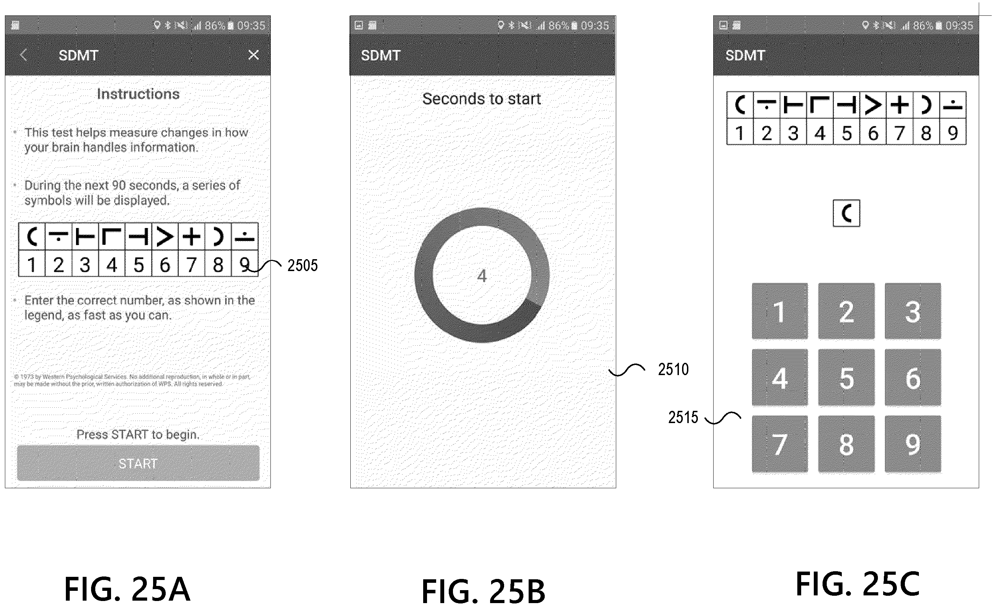

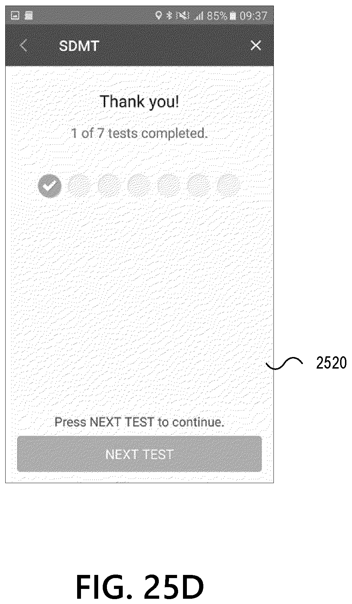

FIGS. 25A-25D depict example screenshots 2505, 2510, 2515, and 2520 illustrating an example SDMT test according to one or more illustrative aspects described herein. As shown in the screenshot 2505 of FIG. 25A, the subject is provided with instructions for the SDMT test. The instructions indicate that the test measures changes in how the subject's brain handles information. The subject 110 is prompted to match symbols with numbers according to a key as quickly and accurately as possible. The key, symbols, numbers are displayed on the display screen 160 of the device 105. Next, as shown in the screenshot 2510 of FIG. 25B, a countdown to the start of the test is displayed. The test begins and as shown in the screenshot 2515 of FIG. 25C, a key, symbols, and numbers are displayed on the display screen 160 of the device 105. The subject matches the symbols with the numbers according to the key as quickly and accurately as possible. Then, as shown in the screenshot 2520 of FIG. 25D, the subject 110 is informed that the test is complete.

In some embodiments, the diagnostic tasks implement a word reading test. The subject is instructed to read aloud color words written in black font on the display screen 160 of the device 105. The voice of the subject 110 is recorded. This test assesses cognitive processing speed, and is modelled on the "Word Reading" part of the Stroop Word Reading (SWR) Test (Ridley 1935). The "Word Reading" part of the SWR Test has been shown to be sensitive to symptom changes in patients with early Huntington's disease (Tabrizi 2012) and is part of the UHDRS assessment (Huntington's Study Group 1996).

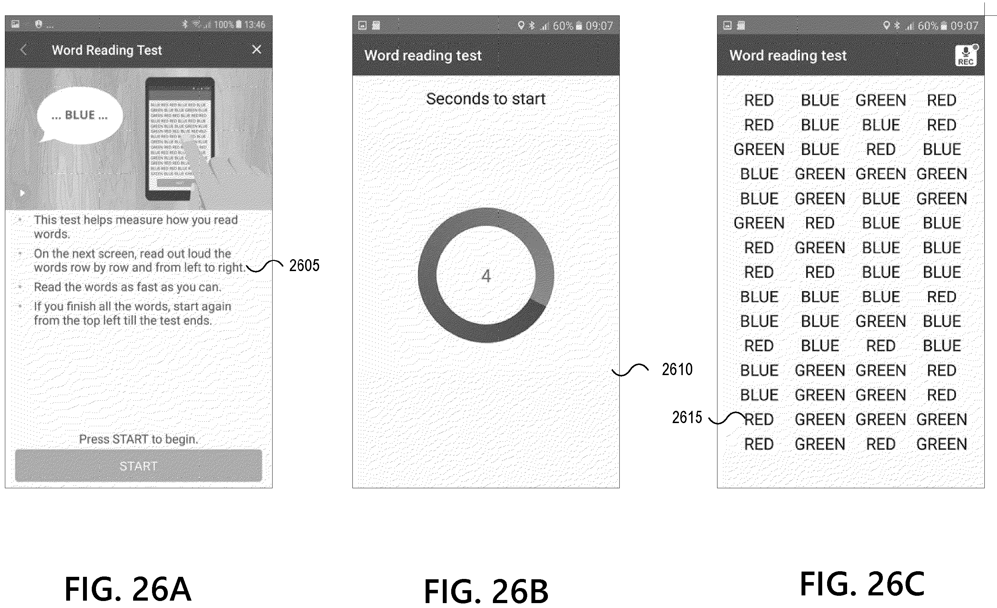

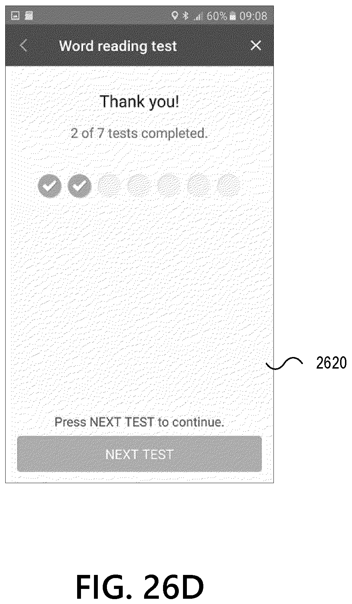

FIGS. 26A-26D depict example screenshots 2605, 2610, 2615, and 2620 illustrating an example word reading test according to one or more illustrative aspects described herein. As shown in the screenshot 2605 of FIG. 26A, instructions for word reading are displayed on the display screen 160 of the device 105. The instructions inform the subject that the test measures how the subject reads words. The subject is instructed to on the next screen, read out loud the words row by row and from left to right as fast as possible. The subject is instructed to start again from the top left unit the test ends if the subject finished reading all the words. Next, as shown in the screenshot 2610 of FIG. 26B, a countdown to the start of the test is displayed. The test begins and as shown in the screenshot 2615 of FIG. 26C, words arranged in rows are displayed on the screen 160 of the device 105. The subject reads the words as instructed. Then, as shown in the screenshot 2620 of FIG. 26D, the subject 110 is informed that the test is complete.

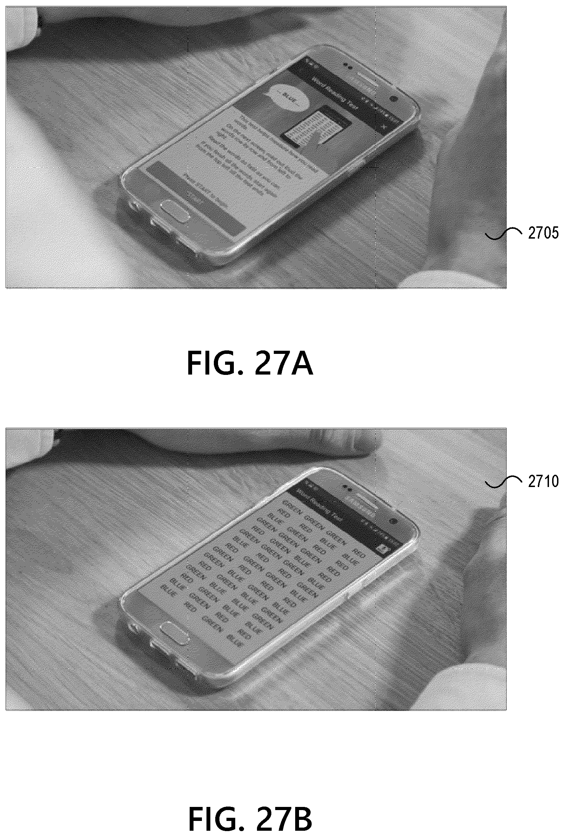

In some embodiments, the diagnostic application may include an instructional video for the word reading test that the subject may view on the display screen 160 of the device 105. FIGS. 27A-27B depict example screenshots 2705 and 2710 from an instructional video for an example word reading test according to some embodiments.

In some embodiments, the diagnostic tasks are automatically scheduled and take about 5 minutes per day. In some embodiments, if the subject 110 does not complete the diagnostic tasks on the scheduled day, the scheduled tasks that occur less frequently than every second day (e.g., EQ-5D-SL, 5 Level Questionnaire, WPAI-HD, HD-SDI, Walk Test) are rolled over to the next time the diagnostic tasks are performed.

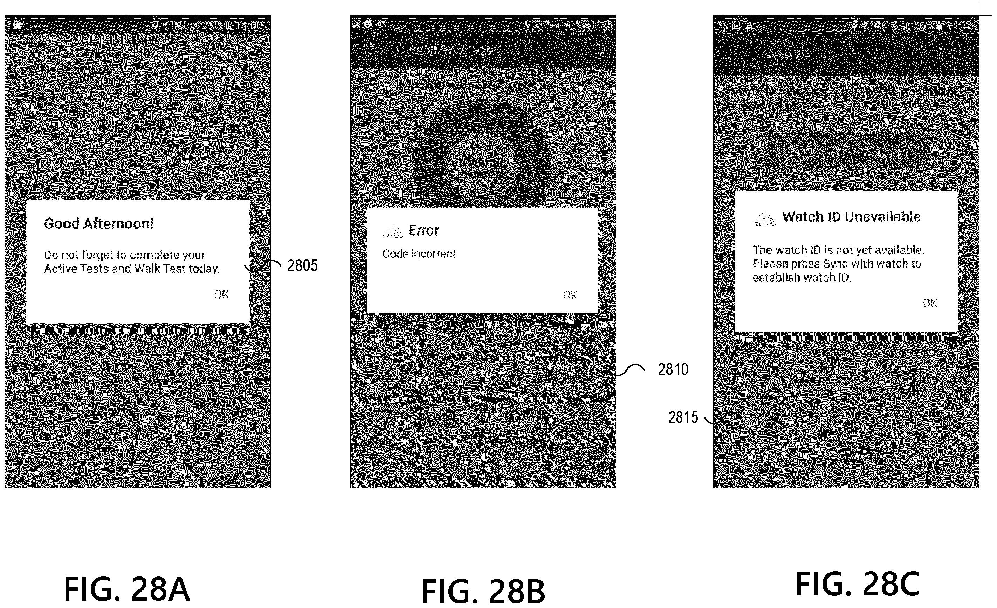

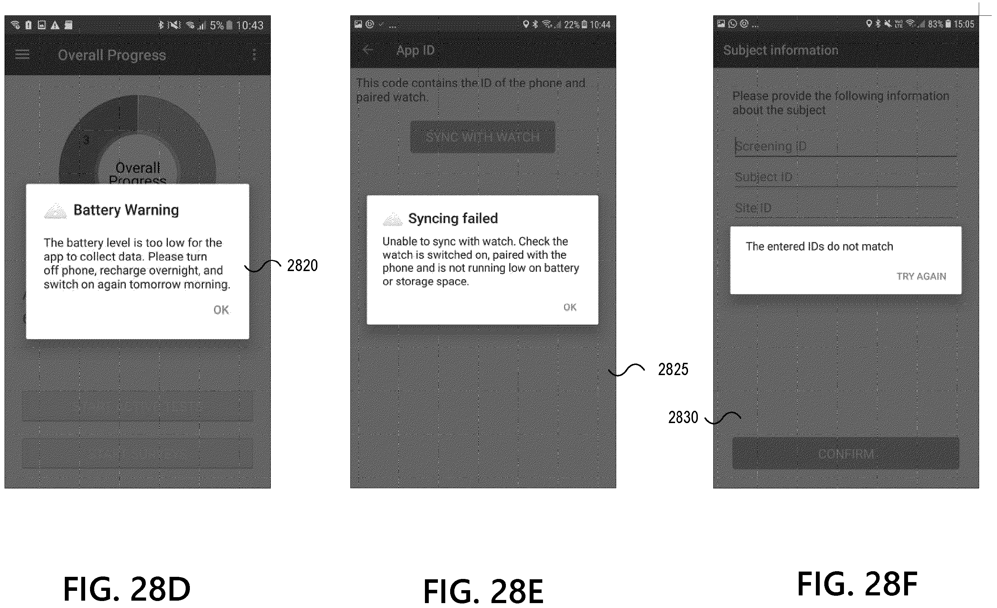

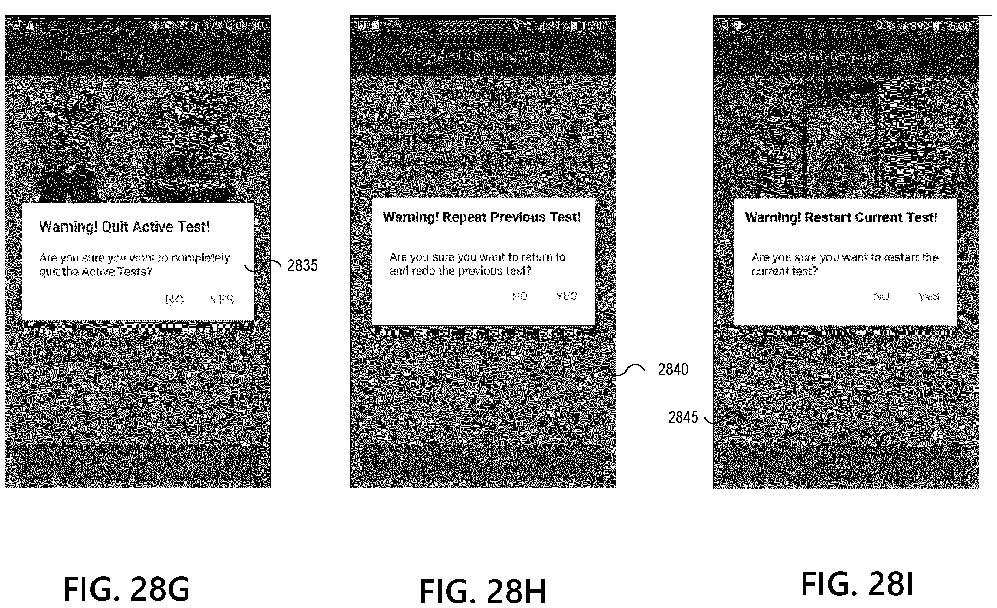

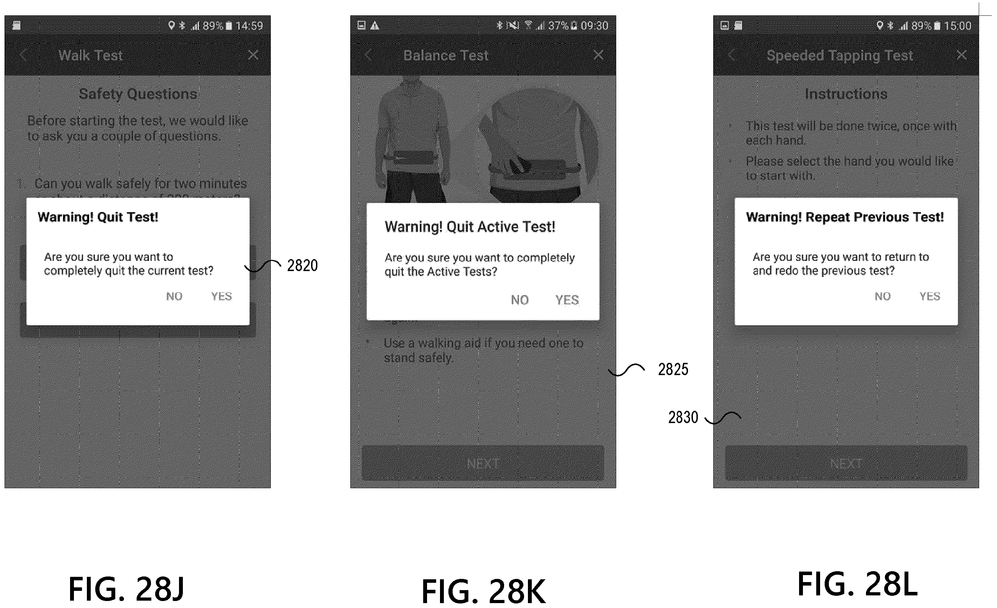

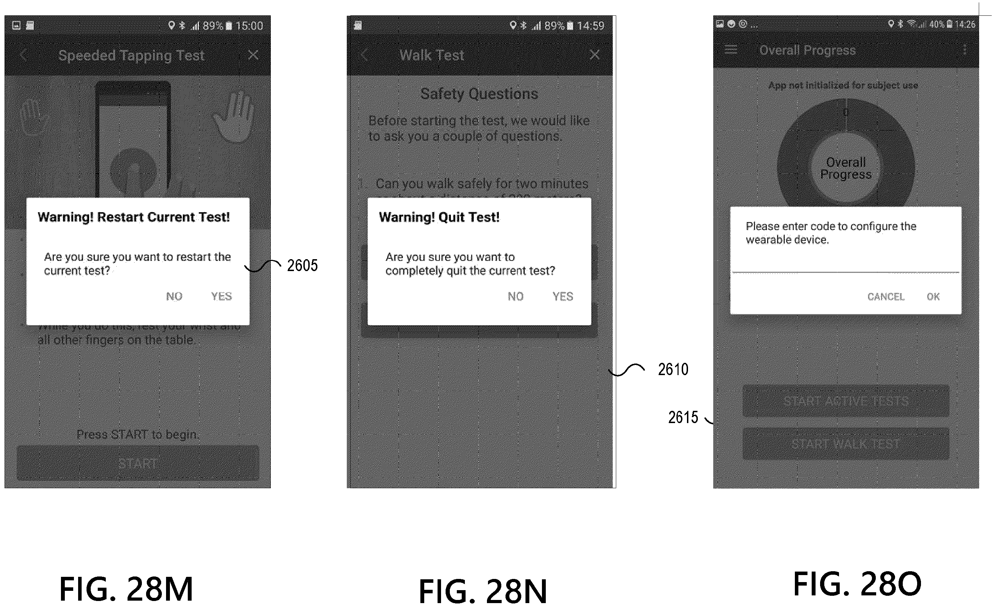

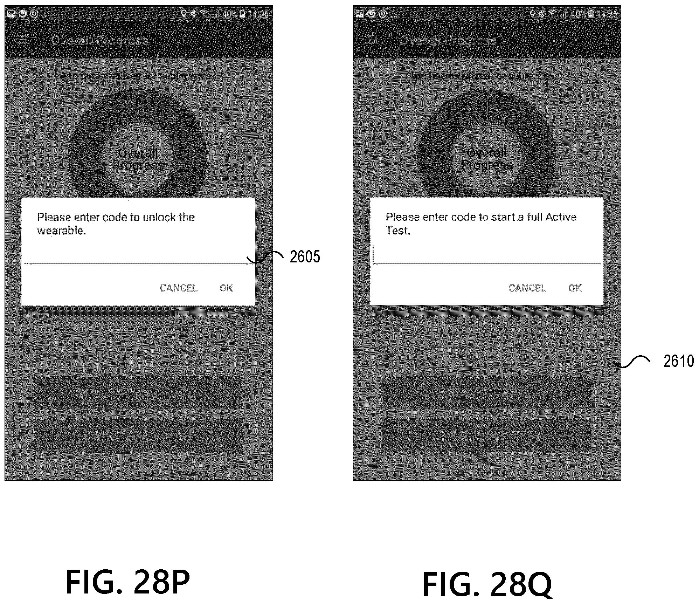

In some embodiments, the diagnostic application may display various messages or warnings unrelated to the active tests. FIGS. 28A-28Q depict example screenshots illustrating the various messages and/or warnings displayed by the diagnostic application according to one or more illustrative aspects described herein.

The method 300 proceeds to step 310 which includes in response to the subject performing the one or more diagnostics tasks, receiving, a plurality of second sensor data via the one or more sensors. In response to the subject 110 performing the one or more diagnostic tasks, the diagnostic device 105 receives, a plurality of sensor data via the one or more sensors associated with the device 105. As mentioned above, the sensors associated with the device 105 include a first sensor 120a that is disposed within the device 105 and a second sensor 120b that is worn by the subject 110. The device 105 receives a plurality of first sensor data via the first sensor 120a and a plurality of second sensor data via the second sensor 120b.

The method 300 proceeds to step 315 including extracting, from the received sensor data, a second plurality of features associated with one or more symptoms of Huntington's disease. The device 105 extracts, from the received first sensor data and second sensor data, features associated with one or more symptoms of Huntington's disease in the subject 110. The symptoms of Huntington's disease in the subject 110 may include a symptom indicative of a cognitive function of the subject 110, a symptom indicative of a motor function of the subject 110, a symptom indicative of a behavioral function of the subject 110, or a symptom indicative of a functional capacity of the subject 110. In some embodiments, the extracted features of the plurality of first and second sensor data may be indicative of symptoms of Huntington's disease such as visuo-motor integration, visual attention, motor speed, cognitive processing speed, chorea, dystonia, visuo-motor coordination, fine motor impairment, upper-body or lower-body bradykinesia. As discussed above, location-based data from a GPS or similar system may be used to assess symptoms related to the motor function and/or mobility of the subject and other location based assessments. Similarly, WiFi and Bluetooth signal density may be used to help assess patent sociability and the like.

The method 300 proceeds to step 320 which includes determining an assessment of the one or more symptoms of Huntington's disease based on at least the extracted sensor data. The device 105 determines an assessment of the one or more symptoms of Huntington's disease in the subject 110 based on the extracted features of the received first and second sensor data. In some embodiments, the device 105 may send the extracted features over a network 180 to a server 150. The server 150 includes at least one processor 155 and a memory 160 storing computer-instructions for a symptom assessment application 170 that, when executed by the processor 155, determine an assessment of the one or more symptoms of Huntington's disease in the subject 110 based on the extracted features received by the server 150 from the device 105. In some embodiments, the symptom assessment application 170 may determine an assessment of the one or more symptoms of Huntington's disease in the subject 110 based on the extracted features of sensor data received from the device 105 and a patient database 175 stored in the memory 160. The patient database 175 may include various clinical data. In some embodiments, the second device may be one or more wearable sensors. In some embodiments, the second device may be any device that includes a motion sensor with an inertial measurement unit (IMU). In some embodiments, the second device may be several devices or sensors. In some embodiments, the patient database 175 may be independent of the server 150. In some embodiments, the server 150 sends the determined assessment of the one or more symptoms of Huntington's disease in the subject 110 to the device 105. In some embodiments, such as in FIG. 1, the device 105 may output an assessment of the one or more symptoms of Huntington's disease on the display 160 of the device 105. In some embodiments, the assessment of the one or more symptoms of Huntington's disease may be communicated to a clinician that may determine individualized therapy for the subject 110 based on the assessment.

As discussed above, assessments of symptom severity and progression of Huntington's disease using diagnostics according to the present disclosure correlate sufficiently with the assessments based on clinical results and may thus replace clinical patient monitoring and testing. Diagnostics according to the present disclosure were studied in a group of Huntington's disease patients. The patients were provided with a smartphone application that included 7 active tests and continuous passive monitoring. The active tests included SDMT, Stroop word reading test, speeded tapping test, chorea test, balance test, U-turn test and two minutes long walk test. Data was collected over a period of two weeks 2 after in clinic screening visits for each patient. During the in-clinic screening visits, UHDRS scores, Symbol Digit Modalities Test (SDMT), Stroop Word Test and demographics related variables were collected for each patient.

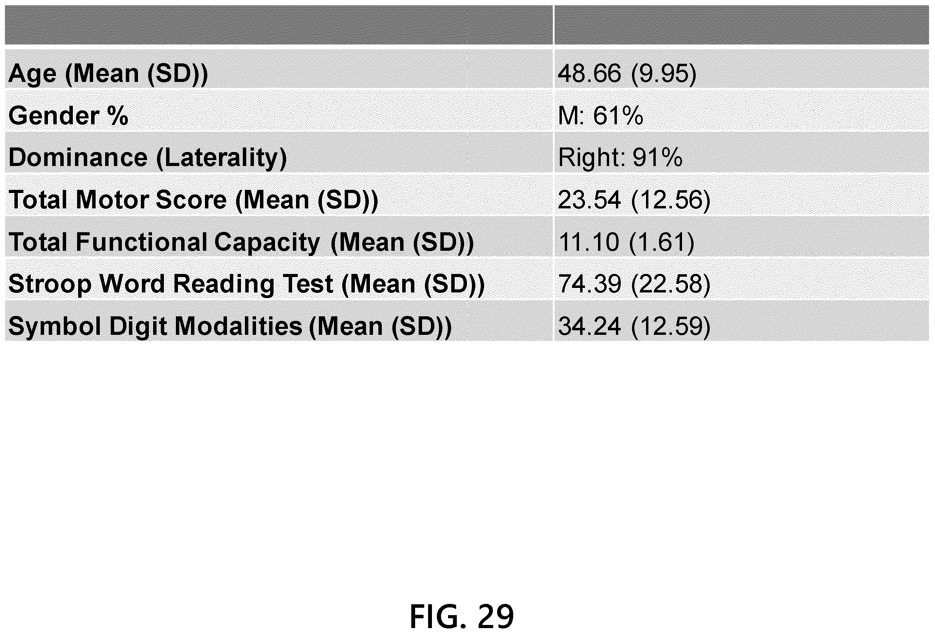

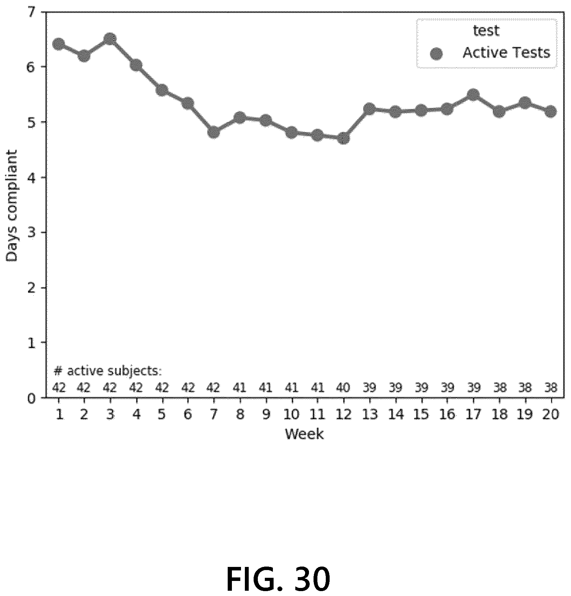

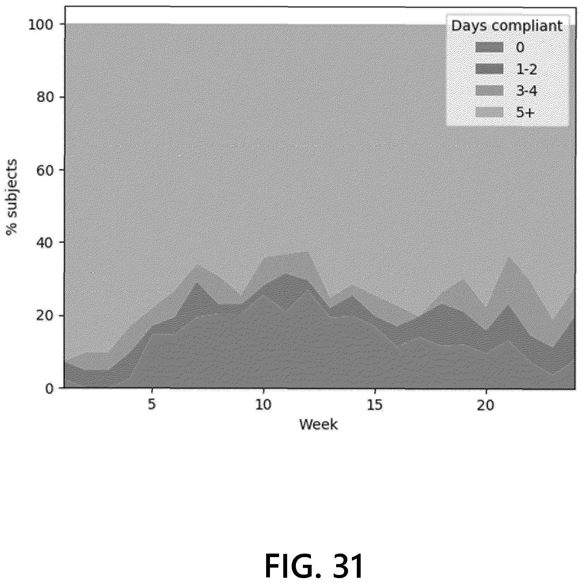

FIG. 29 is a table showing various characteristics of the group of patients participating in a study. FIG. 30 is a graph showing the number of patients completing active tests per week for the first 20 weeks after the in-clinic screening visit. According to FIG. 30, the patients completed, on average, 5 active tests per week. FIG. 31 is a graph showing the number of patients completing active tests per week for the first 20 weeks after the in-clinic screening visit. Additionally, there was no significant influence (all p>0.05) on patient adherence to active testing on various factors such as age and gender, and disease severity (TMS, TFC and Independence Scale).

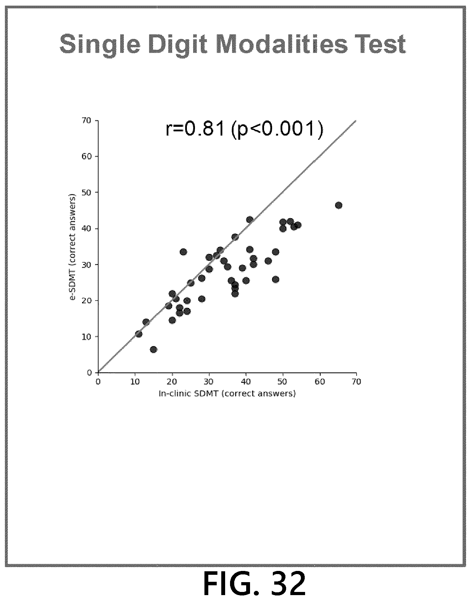

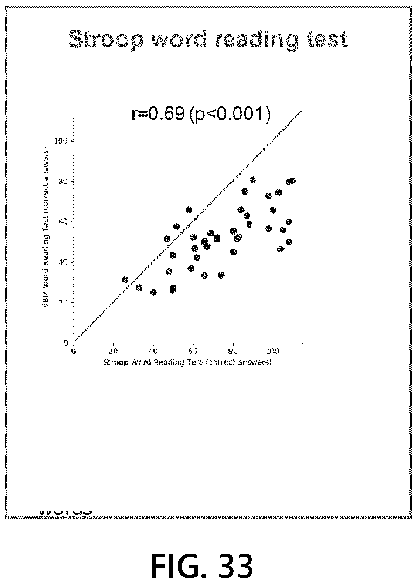

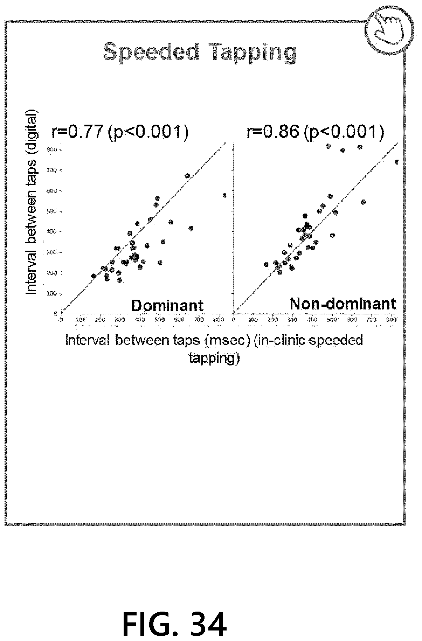

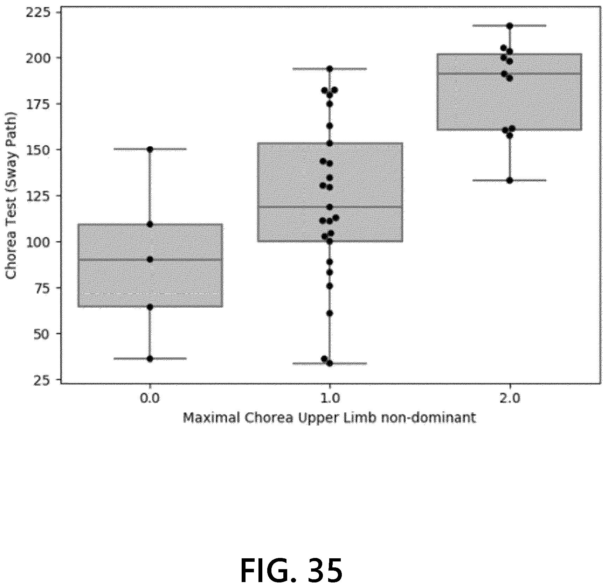

Sensor features were extracted from sensor data measured for each active test and aggregated over two-week periods starting with a baseline clinical visit. Intra-class correlation coefficients and Spearman correlations quantified test-retest reliability and validity compared to equivalent standard in-clinic tests or UHDRS items, respectively. FIG. 32 is a plot showing a correlation between results measured using a smartphone application's SDMT active test and results from standard in-clinic SDMT. FIG. 33 is a plot showing a correlation between results measured using the smartphone application's Stroop word reading active test and results from standard in-clinic Stroop word reading testing. FIG. 34 is a plot showing a correlation between the results measured using the smartphone application's speeded tapping active test and the results measured from standard in-clinic speeded tapping testing. FIG. 35 is a plot showing a correlation between results measured using the smartphone application's chorea test and results from standard in-clinic chorea assessment.

est-retest reliabilities of active tests ranged from 0.74-0.97, median 0.95. Diagnostics according to the present disclosure showed correlations with in-clinic testing ranging from r=0.69 (p<0.001, Stroop word reading) to r=0.86 (p<0.001, Speeded tapping). Active tests anchored to specific UHDRS items showed correlations ranging from r=-0.34 (p=0.03, U-Turn) to r=0.64 (p<0.001, Chorea non-dominant hand).

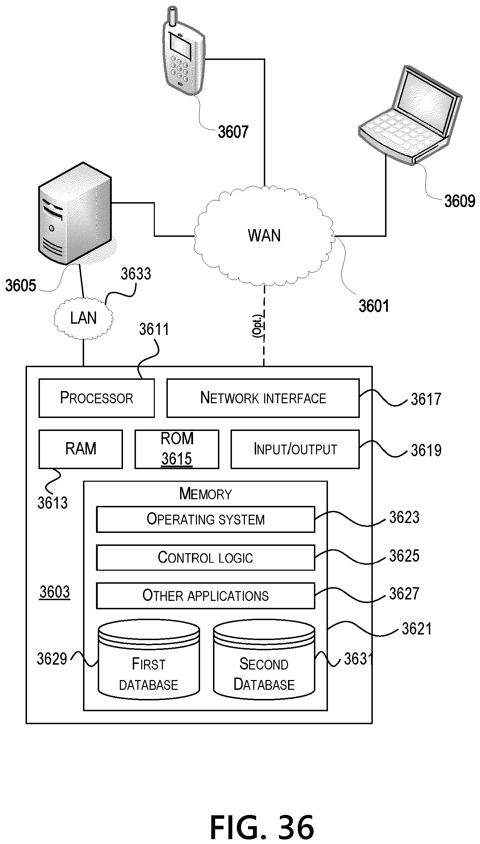

FIG. 36 illustrates one example of a network architecture and data processing device that may be used to implement one or more illustrative aspects described herein, such as the aspects described in FIGS. 1, 2 and 3. Various network nodes 3603, 3605, 3607, and 3609 may be interconnected via a wide area network (WAN) 3601, such as the Internet. Other networks may also or alternatively be used, including private intranets, corporate networks, LANs, wireless networks, personal networks (PAN), and the like. Network 3601 is for illustration purposes and may be replaced with fewer or additional computer networks. A local area network (LAN) may have one or more of any known LAN topology and may use one or more of a variety of different protocols, such as Ethernet. Devices 3603, 3605, 3607, 3609 and other devices (not shown) may be connected to one or more of the networks via twisted pair wires, coaxial cable, fiber optics, radio waves or other communication media.

The term "network" as used herein and depicted in the drawings refers not only to systems in which remote storage devices are coupled together via one or more communication paths, but also to stand-alone devices that may be coupled, from time to time, to such systems that have storage capability. Consequently, the term "network" includes not only a "physical network" but also a "content network," which is comprised of the data-attributable to a single entity-which resides across all physical networks.

The components may include data server 3603, web server 3605, and client computers 3607, 3609. Data server 3603 provides overall access, control and administration of databases and control software for performing one or more illustrative aspects described herein. Data server 3603 may be connected to web server 3605 through which users interact with and obtain data as requested. Alternatively, data server 3603 may act as a web server itself and be directly connected to the Internet. Data server 3603 may be connected to web server 3605 through the network 3601 (e.g., the Internet), via direct or indirect connection, or via some other network. Users may interact with the data server 3603 using remote computers 3607, 3609, e.g., using a web browser to connect to the data server 3603 via one or more externally exposed web sites hosted by web server 3605. Client computers 3607, 3609 may be used in concert with data server 3603 to access data stored therein, or may be used for other purposes. For example, from client device 3607 a user may access web server 3605 using an Internet browser, as is known in the art, or by executing a software application that communicates with web server 3605 and/or data server 3603 over a computer network (such as the Internet). In some embodiments, the client computer 3607 may be a smartphone, smartwatch or other mobile computing device, and may implement a diagnostic device, such as the device 105 shown in FIG. 1. In some embodiments, the data server 3603 may implement a server, such as the server 150 shown in FIG. 1.

Servers and applications may be combined on the same physical machines, and retain separate virtual or logical addresses, or may reside on separate physical machines. FIG. 1 illustrates just one example of a network architecture that may be used, and those of skill in the art will appreciate that the specific network architecture and data processing devices used may vary, and are secondary to the functionality that they provide, as further described herein. For example, services provided by web server 3605 and data server 3603 may be combined on a single server.

Each component 3603, 3605, 3607, 3609 may be any type of known computer, server, or data processing device. Data server 3603, e.g., may include a processor 3611 controlling overall operation of the rate server 3603. Data server 3603 may further include RAM 3613, ROM 3615, network interface 3617, input/output interfaces 3619 (e.g., keyboard, mouse, display, printer, etc.), and memory 3621. I/O 3619 may include a variety of interface units and drives for reading, writing, displaying, and/or printing data or files. Memory 3621 may further store operating system software 3623 for controlling overall operation of the data processing device 3603, control logic 3625 for instructing data server 3603 to perform aspects described herein, and other application software 3627 providing secondary, support, and/or other functionality which may or may not be used in conjunction with other aspects described herein. The control logic may also be referred to herein as the data server software 3625. Functionality of the data server software may refer to operations or decisions made automatically based on rules coded into the control logic, made manually by a user providing input into the system, and/or a combination of automatic processing based on user input (e.g., queries, data updates, etc.).

Memory 3621 may also store data used in performance of one or more aspects described herein, including a first database 3629 and a second database 3631. In some embodiments, the first database may include the second database (e.g., as a separate table, report, etc.). That is, the information can be stored in a single database, or separated into different logical, virtual, or physical databases, depending on system design. Devices 3605, 3607, 3609 may have similar or different architecture as described with respect to device 3603. Those of skill in the art will appreciate that the functionality of data processing device 3603 (or device 3605, 3607, 3609) as described herein may be spread across multiple data processing devices, for example, to distribute processing load across multiple computers, to segregate transactions based on geographic location, user access level, quality of service (QoS), etc.

One or more aspects described herein may be embodied in computer-usable or readable data and/or computer-executable instructions, such as in one or more program modules, executed by one or more computers or other devices as described herein. Generally, program modules include routines, programs, objects, components, data structures, etc. that perform particular tasks or implement particular abstract data types when executed by a processor in a computer or other device. The modules may be written in a source code programming language that is subsequently compiled for execution, or may be written in a scripting language such as (but not limited to) HTML or XML. The computer executable instructions may be stored on a computer readable medium such as a hard disk, optical disk, removable storage media, solid state memory, RAM, etc. As will be appreciated by one of skill in the art, the functionality of the program modules may be combined or distributed as desired in various embodiments. In addition, the functionality may be embodied in whole or in part in firmware or hardware equivalents such as integrated circuits, field programmable gate arrays (FPGA), and the like. Particular data structures may be used to more effectively implement one or more aspects, and such data structures are contemplated within the scope of computer executable instructions and computer-usable data described herein.

Although the subject matter has been described in language specific to structural features and/or methodological acts, it is to be understood that the subject matter defined in the appended claims is not necessarily limited to the specific features or acts described above. Rather, the specific features and acts described above are disclosed as illustrative forms of implementing the claims.

## Claims

1. A diagnostic device (105) for assessing one or more symptoms of Huntington's disease in a subject (110), the device comprising:
at least one processor (115) and a display screen (160);
one or more sensors (120) associated with the device; and
memory (125) storing computer-readable instructions that, when executed by the at least one processor, cause the device to:
receive a plurality of first sensor data via the one or more sensors associated with the device;
extract, from the received first sensor data, a first plurality of features associated with the one or more symptoms of Huntington's disease in the subject; and
determine a first assessment of the one or more symptoms of Huntington's disease based on the extracted first plurality of features,
**characterized in that** the computer-readable instructions, when executed by the at least one processor, further cause the device to:
prompt the subject to perform one or more diagnostic tasks;
in response to the subject performing the one or more diagnostic tasks, receive a plurality of second sensor data via the one or more sensors associated with the device;
extract, from the received second sensor data, a second plurality of features associated with the one or more symptoms of Huntington's disease; and
determine a second assessment of the one or more symptoms of Huntington's disease based on the extracted second plurality of features,
wherein the one or more diagnostic tasks are associated with a chorea test, wherein the subject is prompted to select the hand to start with, the subject is instructed to sit upright and hold the device in the palm of the selected hand with the display screen of the device facing up, the subject is instructed to stretch the corresponding arm out in front and the subject is instructed that when a buzzer of the device vibrates, to close the eyes and count backwards aloud, wherein the subject's voice is recorded, and wherein the test is repeated using the other hand of the subject.

2. The device of claim 1, wherein the one or more symptoms of Huntington's disease in the subject include at least one of a symptom indicative of a cognitive function of the subject, a symptom indicative of a motor function of the subject, a symptom indicative of a behavioral function of the subject, or a symptom indicative of a functional capacity of the subject.

3. The device of any one of claims 1-2, wherein the one or more symptoms of Huntington's disease in the subject include at least one of a symptom indicative of a cognitive function of the subject, a symptom indicative of a motor function of the subject, a symptom indicative of a behavioral function of the subject, or a symptom indicative of a functional capacity of the subject, whereby the patient mobility is assessed at least partly based on GPS location data.

4. The device of any one of claims 1-3, wherein the one or more symptoms of Huntington's disease in the subject are indicative of at least one of visuo-motor integration, visual attention, motor speed, cognitive processing speed, chorea, dystonia, visuo-motor coordination, fine motor impairment, upper-body or lower-body bradykinesia.

5. The device of any one of claims 1-4, wherein the one or more sensors associated with the device comprise at least one of a first sensor disposed within the device or a second sensor worn by the subject and configured to communicate with the device.

6. The device of any one of claims 1-5, wherein prompting the subject to perform the one or more diagnostic tasks includes at least one of prompting the subject to answer one or more questions or prompting the subject to perform one or more actions.
